Europäisches Patentamt

(19) European Patent Office

Office européen des brevets

(11) Publication number: **0 370 719**
**A2**

## EUROPEAN PATENT APPLICATION

(21) Application number: 89311982.6

(22) Date of filing: 20.11.89

(51) Int. Cl.⁵: **C12Q 1/68, C07H 21/04,**
**//C12N15/11**

(30) Priority: 25.11.88 GB 8827544
05.04.89 GB 8907655
05.04.89 GB 8907653

(43) Date of publication of application:
30.05.90 Bulletin 90/22

(84) Designated Contracting States:
AT BE CH DE ES FR GB GR IT LI LU NL SE

(71) Applicant: **IMPERIAL CHEMICAL INDUSTRIES**
**PLC**
**Imperial Chemical House, Millbank**
**London SW1P 3JF(GB)**

(72) Inventor: **Jeffreys, Alec John**
**14 Asquith Boulevard**
**Leicester LE2 6FA(GB)**

(74) Representative: **Mack, John Richard et al**
**Imperial Chemical Industries PLC Legal**
**Department: Patents PO Box 6 Bessemer**
**Road**
**Welwyn Garden City Hertfordshire AL7**
**1HD(GB)**

(54) **Extended nucleotide sequences.**

(57) A method of characterising a genomic DNA sample with reference to at least one informative genetic locus. The method comprises amplifying the minisatellite sequence at at least one informative genetic locus by the use of flanking primers and extension thereof. Novel primers, extension products thereof and diagnostic kits for use in the above method. The method is of particular use for genetic characterisation purposes, paternity and maternity testing as well as forensic testing.

EP 0 370 719 A2

# EXTENDED NUCLEOTIDE SEQUENCES

The present invention relates generally to a method of characterising a genomic DNA sample and to nucleotide sequences employed in the method. In particular the invention involves the use of nucleotide sequences comprising oligonucleotides hybridisable to regions adjacent informative genetic loci. The method of the invention may for example be used in paternity disputes, forensic medicine or in the prevention, diagnosis and treatment of genetic disorders or predispositions. The method is of particular use where only as little as one molecule of an informative locus is present in the genomic DNA sample.

Methods of genetic characterisation are known in the art. In UK patent no. 2166445 (Lister Institute) there are described various DNA sequences which may be used as probes to hybridise simultaneously to a number of polymorphic sites within animal, e.g. human, and plant genomes enabling the production of a "DNA fingerprint" composed of marked DNA bands of differing molecular weights. The DNA fingerprint as a whole is characteristic of the individual concerned and the origin of the differing bands can be traced through the ancestry of the individual and can in certain cases be postulated as associated with certain genetic disorders. In European Patent Application, Publication No. 238329, there are described various DNA sequences which may be used as probes to hybridise individually at individual polymorphic sites within the animal, for example human genome. Methods of genetic characterisation using one or more of such probes are described.

Tandem-repetitive minisatellite regions in vertebrate DNA frequently show high levels of allelic variability in the number of repeat units [1-4]. Hybridization probes capable of detecting multiple minisatellites and producing individual-specific DNA fingerprints have been developed [5-7], as well as cloned human minisatellites which provide locus-specific probes for individual hypervariable loci [5, 8-10]. These highly informative genetic markers have found widespread application in many areas of genetics, including linkage analysis [9, 11-13], determination of kinship in for example paternity and immigration disputes [6, 10, 14, 15], monitoring bone marrow transplants [16,17], and for individual identification in forensic medicine [10, 18, 19]. Applications to typing forensic samples such as blood and semen strains or hair roots are however limited by the sensitivity of the hybridization probes, which require at least 50ng of relatively undegraded human DNA for typing with locus-specific minisatellite probes [10] and 0.01- g DNA for analysis with multilocus DNA fingerprint probes [6].

Where a sufficient amount of sample DNA is available in the test sample the above disclosures provide valuable and reliable methods of genetic characterisation. However the efficiency of the above techniques is reduced where the amount of genomic DNA in the test sample is limited, for example in forensic applications where often only small copy numbers of the test DNA molecule are available.

It is therefore desirable to provide a further method of genetic characterisation which is especially suitable for small samples of genomic DNA.

K. Kleppe et al in J. Mol.Biol. (1971), 56, 341-361 disclose a method for the amplification of a desired DNA sequence. The method involves denaturation of a DNA duplex to form single strands. The denaturation step is carried out in the presence of a sufficiently large excess of two nucleic acid primers which hybridise to regions adjacent the desired DNA sequence. Upon cooling two structures are obtained each containing the full length of the template strand appropriately complexed with primer. DNA polymerase and a sufficient amount of each required nucleoside triphosphate are added whereby two molecules of the original duplex are obtained. The above cycle of denaturation, primer addition and extension are repeated until the appropriate number of copies of the desired DNA sequence is obtained. It is indicated that adjustment of the primer concentration may be required.

The above method is now referred to as polymerase chain reaction (PCR) as claimed in United States patents nos. 4683195 and 4683202 wherein amplification of a given nucleic acid sequence on a template is effected by extension of a nucleic acid primer in the presence of Taq. polymerase or the Klenow fragment of E.coli DNA polymerase I. The amplification procedure is generally repeated for up to about 50 cycles. The examples provided only relate to short DNA sequences, generally of a few hundred base pairs.

The enzymatic amplification of DNA by the polymerase chain reaction (PCR, [20]) enables such smaller amounts of human DNA to be analysed. The remarkable specificity of thermostable Taq polymerase has greatly simplified PCR [21] and has allowed typing of some classes of human DNA polymorphism to be extended to single hair roots [22] and indeed to individual somatic cells and sperm [23]. In most work to date, PCR has been used to amplify short regions of human DNA, usually a few hundred base pairs long [21-23]. Base substitutional polymorphisms can be detected by hybridizing PCR products with allele-specific oligonucleotides [22, 23], by DNA sequence analysis of PCR products [24], or, if the base substitution affects a restriction site, by cleavage of PCR products with a restriction endonuclease [25].

Deletion/insertion polymorphisms can likewise be analysed by sizing PCR products by gel electrophoresis [22]. Most of these marker systems are however dimorphic and their utility in for example forensic medicine is limited by their relatively low variability in human populations.

As explained above PCR has been restricted to the amplification of relatively short DNA sequences and there are serious doubts as to whether PCR may be employed successfully in relation to long DNA sequences especially if faithful reproduction of such sequences is necessary. Indeed 2kb has been stated to represent the absolute limit of PCR [21]. Further difficulties arise however where the sample DNA contains repetitive sequences, for example tandem repeats of a particular core or consensus sequence such as those found at highly informative hypervariable loci.

Thus it can be predicted that hybridisation will take place between amplification products thus resulting in networking and the premature termination of the PCR reaction at an annealed site. Such incomplete PCR products could then act, by out of register annealing to the complementary minisatellite strand, as a substrate for extension in the next cycle of amplification. This will result in the generation of multiple spurious amplification products. The application of PCR to the amplification of minisatellites, particularly long minisatellite alleles containing many repeat units, is thus likely to involve the loss of sequence fidelity and yield such a large number of amplification products that the results of such a process are neither meaningful nor accurately reflect the minisatellite alleles in the starting genomic DNA.

Whilst E. Boerwinkle and L. Chan (1988) in an abstract given out at the 39th Annual Meeting of the American Society of Human Genetics in New Orleans (October, 1988) [Abstract (0548) 12.5] refer to the use of PCR on tandemly repeated hypervariable loci, the size of the targeted region which they employed was relatively small, being always less than one kilobase in length. Moreover they do not disclose the way in which they applied the PCR technique to achieve faithful reproduction of sequences and the production of a meaningful and accurate result. S. Odelberg et al. in a disclosure to the American Academy of Forensic Science (February, 1988) on the other hand have described their unsuccessful application of PCR to larger minisatellites and their findings confirm the predicted difficulties discussed hereinbefore since they are unable to use PCR to obtain any meaningful or useful result in view of the multiplicity of amplification products detected.

The present invention is based on the discovery that as little as one molecule of an informative genetic locus in a genomic DNA sample may be faithfully amplified many times to yield amplification products which are useful for genetic characterisation purposes by effecting the amplification within a defined window such that sufficient of the desired extension product is generated to be detectable but that the yield of extension product is inadequate to permit substantial out-of-register hybridisation between complementary tandem-repeated template strands.

According to one feature of the present invention there is provided a method of characterising a test sample of genomic DNA by reference to one or more controls, which method comprises amplifying the minisatellite sequence at at least one informative locus in the test sample by

(i) hybridising the test sample with primer in respect of each informative locus to be amplified, the primer being hybridisable to a single strand of the test sample at a region which flanks the minisatellite sequence of the informative locus to be amplified under conditions such that an extension product of the primer is synthesised which is complementary to and spans the said minisatellite sequence of the strand of the test sample;

(ii) separating the extension product so formed from the template on which it was synthesised to yield single stranded molecules;

(iii) if required hybridising the primer of step (i) with single stranded molecules obtained according to step (ii) under conditions such that a primer extension product is synthesised from the template of at least one of the single stranded molecules obtained according to step (ii), and

(iv) detecting the amplification products and comparing them with one or more controls; the method being effected such that sufficient of the desired extension product is generated to be detectable but such that the yield of extension product is inadequate to permit substantial out-of-register hybridisation between complementary minisatellite template strands.

It will be appreciated that the cycle of hybridising primer to at least one of the single stranded molecules obtained by separation of the extension product formed from the template on which it was synthesised and then further synthesis of extension product may be repeated as few or as many times as is consistent with the need, on the one hand, to generate sufficient extension product to be detectable and on the other hand to generate a yield of extension product inadequate to permit substantial out-of-register hybridisation between complementary minisatellite template strands.

Where the method of the present invention is effected using one primer in respect of each informative locus it may be advantageous to subject the test sample of genomic DNA to restriction prior to

3

amplification. In this regard where only one primer is used in respect of an informative locus, extension products of varying length are likely to be formed. For example a series of products may be formed having the same 5' terminus, but different 3' termini. A cleaner profile of product is obtainable if the test sample of genomic DNA is subjected to restriction prior to amplification. Where restriction is effected any appropriate restriction endonuclease may be employed. Since the sequence flanking the relevant informative locus will be known and the recognition sequence of known restriction enzymes is also known, the skilled man will encounter no difficulty in selecting an appropriate restriction enzyme for use. Where only one primer is used in respect of each informative locus arithmetical amplification will be achieved, but where two such primers are employed exponential amplification may be obtained. The method of the present invention is thus preferably effected using two primers in respect of each informative locus to be amplified.

Thus according to a further feature of the present invention there is provided a method of characterising a test sample of genomic DNA by reference to one or more controls, which method comprises amplifying the minisatellite sequence at at least one informative locus in the test sample by

(i) hybridising the test sample with two primers in respect of each informative locus to be amplified, each primer being hybridisable to single strands of the test sample at a region which flanks the minisatellite sequence of the informative locus to be amplified under conditions such that an extension product of each primer is synthesised which is complementary to and spans the said minisatellite sequence of each strand of the test sample whereby the extension product synthesised from one primer, when it is separated from its complement, can serve as a template for synthesis of the extension product of the other primer;

(ii) separating the extension product so formed from the template on which it was synthesised to yield single stranded molecules;

(iii) if required hybridising the primers of step (i) with the single stranded molecules obtained according to step (ii) under conditions such that a primer extension product is synthesised from the template of each of the single stranded molecules obtained according to step (ii); and

(iv) detecting the amplification products and comparing them with one or more controls; the method being effected such that sufficient of the desired extension product is generated to be detectable but such that the yield of extension product is inadequate to permit substantial out-of-register hybridisation between complementary minisatellite template strands.

As stated above it will be appreciated that the cycle of hybridising primer to each of the single stranded molecules obtained by separation of the extension product formed from the template on which it was synthesised and then further synthesis of extension product, may be repeated as few or as many times as is consistent with the requirement on the one hand to generate sufficient extension product to be detectable and on the other hand to generate a yield of extension product inadequate to permit substantial out-of-register hybridisation between complementary minisatellite template stands.

An informative locus includes regions of genomic DNA by reference to which individuals can be distinguished. Hypervariable loci, of which there may be over 1000 in the human genome, may be useful as informative loci. The distinguishing power of an informative genetic locus is often referred to in terms of allelic variation or polymorphism. Generally, the greater the degree of allelic variation or polymorphism between individuals the greater the distinguishing power of the locus in question. As a convenient guide informative genetic loci include those in which at least 3 different alleles can be distinguished in any sample of 100 randomly selected unrelated individuals. It will be appreciated that the term individual has been used above to refer not only to humans, but also to other animals as well as to plants and to cell lines derived from such humans, animals and plants. In each case, however the sample of randomly selected unrelated individuals will all be from the same species.

In respect of the human applications of the present invention the expression "informative genetic locus" as used herein may alternatively be defined as one at which at least 3 different alleles can be distinguished in DNA extracted from any 20 cell lines selected from the following, which cell lines have been deposited with the American Type Culture Collection (ATCC):-

| Cell line | ATCC Deposit No. |
| --- | --- |
| Hela | CCL2 |
| RPMI 2650 | CCL 30 |
| Detroit 532 | CCL 54 |
| Detroit 525 | CCL 65 |
| Detroit 529 | CCL 66 |
| Detroit 510 | CCL 72 |
| WI-38 | CCL 75 |
| Citrullinemia | CCL 76 |

| Cell line | ATCC Deposit No. |
|---|---|
| EB-3 | CCL 85 |
| RAJI | CCL 83 |
| JIYOYE (P-2003) | CCL 87 |
| WI-26 | CCL 95 |
| Detroit 551 | CCL 110 |
| RPMI 6666 | CCL 113 |
| RPMI 7666 | CCL 114 |
| CCRF-CEM | CCL 119 |
| CCRF-SB | CCL 120 |
| HT-1080 | CCL 121 |
| HG 261 | CCL 122 |
| CHP3 (M.W.) | CCL 132 |
| LL47 (MaDo) | CCL 135 |
| HEL 299 | CCL 137 |
| LL 24 | CCL 151 |
| HFLI | CCL 153 |
| WI-1003 | CCL 154 |
| MRC-5 | CCL 171 |
| IMR-90 | CCL 186 |
| LS 174T | CCL 188 |
| LL 86(LeSa) | CCL 190 |
| LL 97A (AIMy) | CCL 191 |
| HLF-a | CCL 199 |
| CCD-13Lu | CCL 200 |
| CCD-8Lu | CCL 201 |
| CCD-11Lu | CCL 202 |
| CCD-14Br | CCL 203 |
| CCD-16Lu | CCL 204 |
| CCD-18Lu | CCL 205 |
| CCD-19Lu | CCL 210 |
| Hs888Lu | CCL 21 |
| MRC-9 | CCL 212 |
| Daudi | CCL 213 |
| CCD-25Lu | CCL 215 |

6

| Cell line | ATCC Deposit No. |
|-----------|------------------|
| SW403 | CCL 230 |
| NAMALWA | CRL 1432 |

All the above-mentioned cell lines are freely available from the ATCC, 12301 Parklawn Drive, Rockville, Maryland 20852-1776, USA and are listed in the ATCC catalogue of Cell Lines and Hybridomas. All the above-mentioned cell lines were on deposit with the ATCC prior to 1985.

Convenient informative loci for use in the present invention include loci to which nucleotide sequences and probes disclosed in European patent application no. 238329 are capable of hybridisation, as well as those informative loci the flanking sequences of which are disclosed in this application. Further informative loci include genomic DNA regions identified by minisatellite probes disclosed by S J Gendler et al. in PNAS, 84, (1987), pages 6060-6064. A particularly informative locus is the 5' alpha globin HVA disclosed by the American Journal of Human Genetics, 1988, 43, pages 249-256.

Convenient informative loci for use in the present invention include those of up to 15 kilobases. More convenient informative loci include those which provide short alleles, the upper limit being for example of less than 10 kilobases particularly of less than 8, more particularly less than 6 kilobases. Conveniently the alleles are of at least 1, particularly at least 1.5, more particularly at least 2 kilobases. Thus suitable ranges include 1, 1.5, 2, 2.5, or 3, to 6, 7, 8, 9 or 10 kilobases. In respect of the performance of the method of the invention starting with only a single sample DNA molecule it is preferred that the length of the alleles does not exceed 6 kilobases. Also preferred are informative loci with a restricted range of allele length. It has been observed that these preferences are not always compatible with highly informative loci since these are usually associated with large numbers of minisatellite repeat units and long alleles. The skilled man will however have no difficulty in selecting convenient loci for his purpose.

Primers capable of hybridisation to regions of sample DNA flanking informative loci are required to provide points for the initiation of synthesis of extension products across informative genetic loci. Such primers are generally oligonucleotides and are preferably single stranded for maximum efficiency in amplification, but may alternatively be double stranded. If double stranded, the primer is first treated to separate its strands before being used to prepare extension polynucleotides. Primers must be sufficiently long to prime the synthesis of extension polynucleotides. The exact length of such primers will depend on many factors including temperature and the nature of the primer. Generally a primer will comprise at least seven nucleotides, such as 15-25 nucleotides, for example 20-25 nucleotides. It will be appreciated that the flanking sequence need not reflect the exact sequence of the flanking region of the informative locus. It is merely necessary that the respective sequences are homologous to a degree enabling hybridisation. The extension product so obtained must also be capable of acting as a template for further hybridisation with a primer. The maximum length of any primer is not believed to be critical and is only limited by practical considerations.

Normally the test sample DNA will be double stranded and it is therefore convenient to select primers which hybridise to different strands of the sample DNA and at relative positions along the sequence such that an extension product synthesised from one primer can act as a template for extension of the other primer into a nucleic acid of defined length.

The flanking regions of certain of the informative genetic regions identified by the probes disclosed in UK Patent No. 2166445 and European patent application no. 238329 referred to above and nucleotide sequences hybridisable thereto have not been previously disclosed and both individually and in any combination represent further aspects of the present invention. The sequences are set out on pages preceding the Examples. The informative genetic regions are indicated on some sequences by the word MINISATELLITE. As explained above any convenient sequence may be used as a primer. Examples of some convenient primer sequences have been underlined. For clarity and brevity, on some sequences only the outermost repeat unit on each side of each minisatellite tandem array is shown (in capitals), separated by a series of x's to indicate the omission of most repeats. Since there is seldom a whole number of repeat units in each array, these outermost repeats may be out of register with each other, but preserve the correct relation to the immediate flanking DNA.

A previously unsuspected tandem repeat region was found by sequencing pMS31 (EP-238329), this array is separated from the major minisatellite by 10 bases, and consists of more than 7 repeats of a 19bp G/C rich sequence which extends to the Sau3AI site defining the end of the clone. This region, designated 31B, is G-rich on the opposite strand to the G-rich strand of the major minisatellite. Genomic mapping shows that, if variable at all, 31B makes a minimal contribution to the length variation at this locus (data not shown).

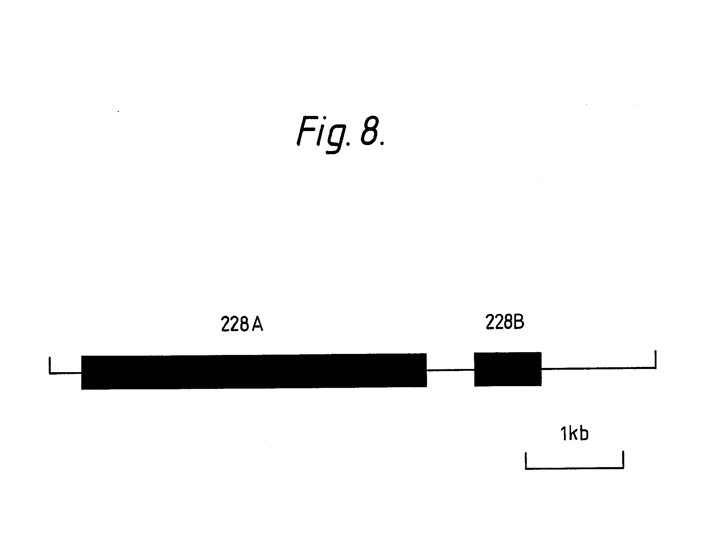

Two loci were detected by pMS228 (Armour et al, 989, NAR, 17, 13, 4925-4935) at high stringency. Family analyses showed these loci to be tightly linked. Restriction mapping of the cloned DNA, subsequently confirmed by sequence analysis, demonstrated the presence of two distinct tandem repetitive regions, designated 228A and 228B (Figure 8). Reprobing human DNA with subclones from pMS228 showed that 228A detected the larger, more intensely hybridising locus, and 228B the smaller, more faintly hybridising alleles. These ministallites were localised, using somatic cell hybrids and in situ hybridisation, to 17p13-pter. The properties of the loci detected by pMS51 and pMS228, and by other minisatellites are summarised in Table 2.

Previous evidence for the disposition of a pair of minisatellites in PMS43 as descrobed in our UK patent application 8813781.5 was confirmed by sequence data, and two further examples (pMS31 and pMS228) of minisatellite clones containing two closely adjacent tandem repeat arrays were elucidated. Unlike pMS43, in which the 'minor' locus 43B has a low (30%) heterozygosity, both the minisatellites in pMS228 have heterozygosities greater than 80%. The minisatellite 228B (figure 8, table 2) combines a high heterozygosity (85%) with a limited allele size range (0.6 -5.5kb); this combination makes it a very useful locus for analysis of minisatellites by the polymerase chain reaction [23]. In general, the most variable loci have a wide range of allele sizes such that many alleles will exceed the maximum size currently amplifiable by this method, so giving an incomplete profile. At the locus detected by 228B, in contrast, a survey of 48 unrelated people showed that 95% of alleles were smaller than 2kb, and that the largest (5.5kb) was well within the amplifiable range (J Armour and A Jeffreys, unpublished). We have shown that even the largest allele at this locus can be amplified, providing the prospect of a complete and yet usefully informative profile from amplification at a single locus.

A novel cloned minisatellite termed MS29 has been isolated which unusually detects two variable loci in human DNA. One locus, located in the terminal region of the short arm of human chromosome 6, is also present in great apes. The second minisatellite locus is located interstitially on chromosome 16p11, and is absent both from non-human primates and from some humans. MS29 was isolated from a L47 genomic library made from DNA enriched for minisatellite sequences as described elsewhere (Wong et al, Ann.Hum.Genet. (1987) 51, 260-288. MS29 comprises a 39bp repeat sequence. This consists of 13 diverged repeats of the trinucleotide YAG wherein Y represents any one of A, G, C or T. The flanking sequences of this minisatellite can be elucidated using sequencing techniques known per se.

In summary the relevant novel flanking sequences are those of MS1, MS29, MS31A and MS31B, MS32, MS43A and MS43B, MS51, MS228A and MS228B. In addition we now also provide further novel flanking sequence information for the minisatellite probe p g3 (Wong et al, Nucleic Acids Research 14, 11, (1986), 4605-4616). as well as for the probes 33.1, 33.4 and 33.6 (UK Patent 2166445/The Lister Institute of Preventive Medicine).

A particular group of flanking sequences are those of the minisatellite probes MS1, MS29, MS31A and MS31B, MS32, MS43A and MS43B, MS51, MS228A and MS228B.

Further particular groups of flanking sequences are comprised by those of any of the above minisatellite probes.

As stated previously the minisatellites MS29 and MS31B are novel and the repeat sequences and/or flanking sequences of either of these minisatellites represent further particular aspects of the invention.

The method of the present invention can be used in respect of any convenient informative locus. Thus, for example primers may be prepared for hybridisation to the flanking sequences of for example the hypervariable region 5′ to the human insulin gene (Am.J.Hum Genet, 1986, 39, 291-229), of the 5′ alpha globin HVR (Am.J.Hum. Genet, 1988, 43, 249-256), of the alpha globin 3′ HVR (EMBOJ, 1986, 5, 1957-1863), of the hypervariable region at the zeta globin locus (PNAS, 1983, 80, 5022-5026), or of the Ha Ras locus (Nature, 1983,302 33-37). Also incorporated by reference in this application are a panel of probes proposed by Ray White. The flanking sequences of these may be elucidated using known techniques.

The extended flanking polynucleotides prepared according to the method of the invention represent a further aspect of the invention. These polynucleotides may be present in single or multiple copies. Where multiple copies are present these are faithful copies and preferably substantially free from networking or cross linkage between individual strands. By the term faithful we mean that the genetic characterisation information to be obtained from the size and composition of the copy is essentially the same for all copies. Conveniently the extended primers comprise a sequence identical with or complementary to an informative genetic locus of more than 1 kilobase. Other convenient values include 1.5 and 2 kilobases. Conveniently the number of copies represent the products of at least 3, 5, 7, 9, 13 or 15 cycles of the amplification method of the present invention.

According to a still further aspect of the invention there is provided a mixture containing multiple faithful copies of extended primers (as hereinbefore defined). As above the mixture is substantially free from networking or cross-linkage between individual strands.

The primers for use in the present invention may be prepared by methods analogous to those known in the art. For example where a given flanking sequence is known, a convenient nucleotide primer may be prepared by direct synthesis. Cloning techniques may also be used to reproduce DNA fragments containing sequences flanking informative genetic loci.

Alternatively DNA fragments comprising informative genetic loci may be identified and using a procedure analogous to that of the methods outlined above, a nucleotide sequence hybridisable to the informative genetic locus may be extended. The product so obtained may then be directly modified, for example by cleavage, to prepare a convenient primer or the sequence thereof may be determined and convenient primers then prepared by direct synthesis.

As explained above the sample DNA is generally double stranded. It is therefore desirable to separate the strands of the nucleic acid before it can act as a template for extension of a primer. Strand separation can be accomplished by any suitable method including physical, chemical or enzymatic means. Conveniently heat denaturation is used to provide up to about 99% denaturation. Typical temperatures used include 85-105°C for times ranging from about 1-10 minutes.

A primer is preferably used for each unique strand of the sample DNA. In respect of double stranded DNA two primers are therefore normally used. Generally the primers are chosen to allow extension to proceed across the informative locus in the same direction along both strands, that is to say 5' to 3' or vice versa. Preferably extension proceeds 5' to 3'.

Primers are conveniently hybridised with the genomic DNA sample under known conditions. Generally this is allowed to take place in a buffered aqueous solution, preferably at a pH of 7-9. Preferably a large excess of primer is present in the reaction mixture. It will be appreciated that in certain diagnostic applications the amount of sample DNA may not be known but an excess of primers is desirable.

The amplification is conveniently effected by adding the deoxyribonucleoside triphosphates dATP, dCTP, dGTP, and dTTP to the synthesis mixture in adequate amounts and the resulting solution may then, for example be heated to for example about 90°-100°C for a period of for example from about 1 to 10 minutes. After this heating period the solution may be allowed to cool to 30-70°C which is preferable for the primer hybridisation. Where Taq polymerase is used as the inducing agent to facilitate the extension reaction it is preferable to effect primer hybridisation at a temperature of from 50-70°C for example 50°-65°C, such as 60°C.

It has been found that primer hybridisation is advantageously effected in a buffer of reduced ionic strength and at an elevated annealing temperature. This reduces the possibility of mispriming. The expressions "reduced ionic strength" and "elevated annealing temperature" are to be understood as referring to conditions at the extremes of, or outside those ranges which would generally be considered appropriate by the molecular biologist of ordinary skill for a given nucleic acid amplification reaction. By way of illustration, but not limitation a convenient buffer will be of sufficient ionic strength to enable a stable pH of 7-9 to be maintained. Convenient annealing temperatures are generally 50-60°C. It will be appreciated that the actual reaction conditions will depend on the primer(s) used.

An appropriate agent for inducing or catalysing the extension reaction may then be added to the annealed mixture and the reaction may then be allowed to proceed under conditions known in the art.

The inducing agent is conveniently an enzyme. Suitable enzymes include the Klenow fragment of E. coli DNA polymerase I, T4 DNA polymerase, but particularly Taq polymerase. Other available DNA polymerases, reverse transcriptase and other enzymes, including heat stable enzymes which will facilitate the extension reaction may also be used.

The newly synthesised strand and its complementary nucleic acid strand form a double-stranded molecule which is used in the succeeding steps of the method. The steps of strand separation and sequence extension may then be repeated as required using the procedures outlined above. Suitable conditions for such procedures are outlined in US patent nos. 4683202 and 4683195 incorporated by reference above. As explained in the above patent specifications the separation and extension cycles may be performed stepwise or advantageously a plurality of cycles are operated for example in a semi-automated or fully automated manner.

In the conventional PCR technique the amplification reaction is generally allowed to proceed for up to about 50 cycles. In contrast, acccording to the method of the present invention the upper limit for amplification is believed to be about 25 cycles when starting from a single copy of the sample DNA. Where larger starting amounts of genomic DNA sample are available fewer cycles of amplification are required. The convenient window of amplification cycles depending on the amount of sample DNA and the nature of the sequence will now be illustrated, but in no way limited, by reference to the following analysis:

The following analysis refers to amplification reactions using $10\mu l$ volumes and, with extension times of 15 minutes: Considering an individual heterozygous for alleles A and B, where A is longer than B it can be predicted that A will therefore amplify less efficiently than B. The lower limit of cycle number $C_1$ is dictated by the sensitivity of the probes, which can detect about 0.1 pg product. To detect allele A, sufficient cycles are needed to generate >0.1 pg allele A product. Similarly, >0.1 pg allele B must be produced to detect both alleles. The upper limit of cycle number $C_u$ is limited by the yield of both alleles A and B. $C_1$ and $C_u$ can be estimated as follows:

Let M = initial mass of human genomic DNA (in picograms).

a = length of allele A (kb)

b = length of allele B (kb)

ga = gain per PCR cycle of allele A

gb = gain per PCR cycle of allele B

Since the diploid genome size of man is $6 \times 10^6$ kb then the initial amount of allele A is

$$M . \frac{a}{6 \times 10^6} \quad pg$$

Similarly, the intial amount of allele B is

$$M . \frac{b}{6 \times 10^6} \quad pg$$

The yield of allele A after C cycles is given by:

$$M . \frac{a}{6 \times 10^6} \quad g_a{}^c \quad pg$$

and similarly for allele B.

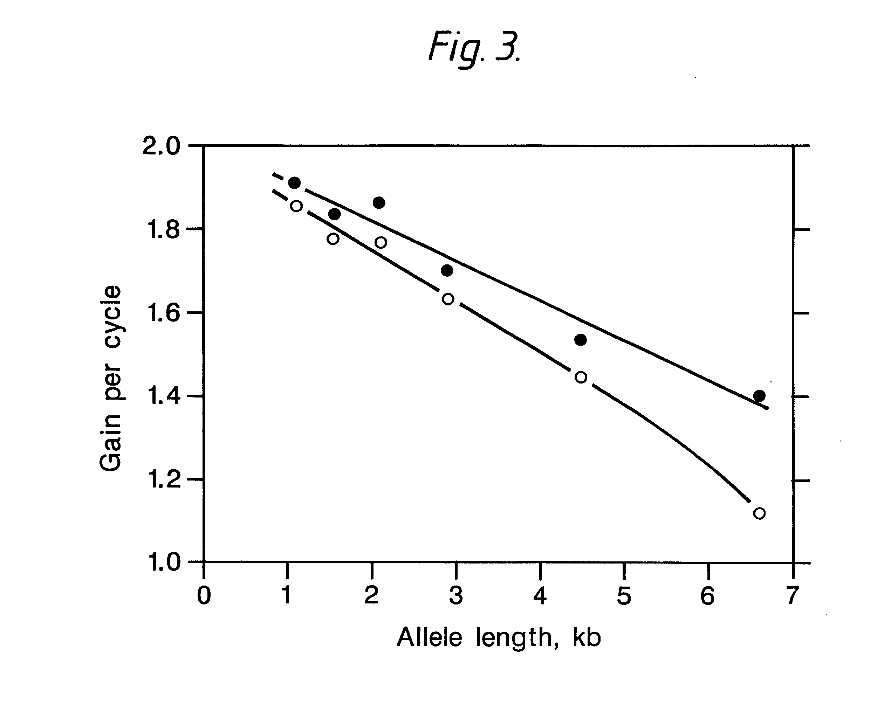

From Fig. 3 the gain per cycle g is related to allele length L by

$gL \equiv 2 - 0.093 \, L \; (L = 0\text{-}6 \text{ kb})$

The lower limit of cycles $C_1$ is therefore set by the lowest value of C where

$$\frac{M.a}{6 \times 10^6} \quad (2 - 0.093.L)^c \; > \; 0.1$$

and

$$\frac{M.b}{6 \times 10^6} (2 - 0.093.L)^c \; > \; 0.1$$

The upper limit $C_u$ is likewise set by the highest value of C where the total yield of both alleles is < 1000 pg, i.e.

$$\frac{M.a}{6 \times 10^6} \quad (2 - 0.093.L)^c \; + \; \frac{M.b}{6 \times 10^6} \quad (2 - 0.093.L)^c \quad <1000$$

$C_1$ and $C_u$ can be determined by computer reiteration. Some typical examples are:

| M | a | b | $C_l$ | $C_u$ |
|---|---|---|---|---|
| pg | kb | kb | | |
| 10,000 | 5 | 1 | 7 | 20 |
| 1,000 | 3 | 2 | 10 | 24 |
| 100 | 6 | 0.5 | 19 | 27 |
| * 6 | 6 | 1 | $27^+$ | $32^+$ |

+ note that the window becomes very narrow and can disappear for very small amounts of DNA with widely differing allele sizes.
* N.B. This model does not take into account the fact that significant stochastic variation in the number of target molecules exists in amounts of human genomic DNA <100pg (equivalent to 17 cells). The analysis for 6 pg DNA corres onds to the amplification of a single target molecule of each allele.

Homozygotes can be readily accomodated in the model. $C_u$ remains unaltered, whereas $C_l$ is defined by the number of cycles required to give >0.05 pg of each allele (i.e. >0.1 pg combined).

Reactions with different volumes can also be accomodated. $C_l$ is defined by the number of cycles required to give >0.1 pg of each allele in the total reaction. $C_u$ is defined by the number of cycles required to give <1000 pg total product per $10\mu l$ of reaction. For large volume PCR reaction, $C_l$ will be unchanged but $C_u$ will be somewhat larger than with a $10\mu l$ reaction.

The window of amplification cycles which generates an appropriate ammount of amplification product for characterisation (0.1-4000 pg of product) is for example 10-15 cycles for 100 ng of genomic DNA, 18 cycles for 1ng and 25 cycles for single cell amplification (6pg). The number of PCR cycles may need to be increased to detect larger alleles which amplify less efficiently. Depending on allele length $1000$-$10^6$ pg of faithfully amplified product can be obtained.

A particular advantage of the claimed method is that differences of as little as one repeat unit in an informative region may be identified. The method of the present invention is also believed to offer faithful reproduction of large informative genetic loci, for example those of up to 15kb, such as up to about 10kb.

It has also been found that the problems associated with the networking of amplified copies of sample DNA as well as the generation of incomplete extension products can lead to the appearance of significant amounts of single stranded minisatellite DNA of heterogeneous size. This single stranded DNA can produce significant levels of aspecific products in the PCR reaction. This problem may be overcome or at least alleviated by the use of enzymes which specifically digest or degrade single stranded DNA and which leave double stranded DNA intact. The preferred enzyme is S1 nuclease. Digestion of the final PCR products with such an enzyme results in a cleaner profile of PCR products revealed at the final detection step. Therefore in a preferred aspect of the present invention the method of the invention includes the use of at least one enzyme which specifically digests or degrades single stranded DNA whilst leaving double stranded DNA intact, whereby to ameliorate the problems of aspecific products of the PCR reaction.

Detection of the amplified products may be effected by any convenient means. The amplified products may be identified and characterised, for example using gel electrophoresis and followed if desired by hybridisation with probes hybridisable to the informative loci contained therein followed by for example autoradiography where radiolabelled probes are used. Convenient procedures are disclosed in European Patent Application, Publication No. 238329. Alternatively more direct methods may be used. Separation of the amplification products on a gel may be followed by direct visualisation thereof. Direct staining with for example ethidium bromide may be effected where a sufficient quantity of product is available. The flanking nucleotide sequences may alternatively carry a label or marker component and such label or marker may then be detected using any convenient method. Such labels or markers may include either radioactive and non radioactive components but the latter are preferred.

The number of informative regions which can be amplified simultaneously is not believed to be limited, other than by practical limitations. For example up to twenty regions could be amplified simultaneously, conveniently ten regions and in particular up to eight, seven, six, five or four regions. In a preferred aspect six regions are amplified simultaneously.

The flanking polynucleotides of the present invention may conveniently be provided in a diagnostic kit. A further aspect of the invention therefore relates to a kit comprising two complementary flanking polynucleotides capable of hybridisation to sample DNA regions adjacent an informative locus together with a control sample of DNA and instructions for their use. Examples of convenient and preferred probes include those outlined elsewhere in the specification. The kit may also include reagent nucleotides for extension of the flanking polynucleotides across the informative genetic locus and/or extension promotor such as an enzyme.

Examples of flanking sequences to informative genetic regions which may be used in the method of the present invention are set out on the following pages. Also disclosed are details of a panel of single locus probes which are capable of hybridisation to informative genetic regions. This panel of probes was disclosed by Ray White at a meeting in Quantico, Virginia in May 1988. Polynucleotide primers capable of hybridisation to regions adjacent to such informative genetic regions may be prepared as previously outlined and used in the method of the invention. Primers may be prepared in respect of any one such region or combination of regions to provide extension products of any desired panel of informative region-(s).

## pMS1.1b  5'flanking sequence extending into minisatellite.

```
5'  GCCATTTCCATAAACACGTATCGAGTACCTAATACTACAAAGTACCATACCAGGTACTAC
    ———————|———————|———————|———————|———————|———————|
3'  CGGTAAAGGTATTTGTGCATAGCTCATGGATTATGATGTTTCATGGTATGGTCCATGATG

    ATCCATTAAGAATGTTAATAATTAATCACGCTCATTTGCCATTGATTTTAAGTTTCCATT
    ———————|———————|———————|———————|———————|———————|
    TAGGTAATTCTTACAATTATTAATTAGTGCGAGTAAACGGTAACTAAAATTCAAAGGTAA

    TTATTAGTGTCAGGTGGTTTTACCAATGGCCTCCCTTTCCTTCATGCTTACACTAAAAAA
    ———————|———————|———————|———————|———————|———————|
    AATAATCACAGTCCACCAAAATGGTTACCGGAGGGAAAGGAAGTACGAATGTGATTTTTT

    AGAACAACAGTAATGATTGTATGTCATGCTTTTCTGTGATGAGCCTTGATGTTTTTAACA
    ———————|———————|———————|———————|———————|———————|
    TCTTGTTGTCATTACTAACATACAGTACGAAAAGACACTACTCGGAACTACAAAAAATTGT

    GAATTTGATAGTTTGAGACATAAAAATTTTTGAAAAACCAACTCACGTTTCCAAACAAAA
    ———————|———————|———————|———————|———————|———————|
    CTTAAACTATCAAACTCTGTATTTTTAAAAACTTTTTGGTTGAGTGCAAAGGTTTGTTTT

    GTGAAAAGGAGAGCTGGATTCCAGCCCCGCCACCCCAGCAAATTGAGAAATCACCCCTTG
    ———————|———————|———————|———————|———————|———————|
    CACTTTTCCTCTCGACCTAAGGTCGGGGCGGTGGGGTCGTTTAACTCTTTAGTGGGGAAC

    CTGTGAGTCAGTGGGT 3'
    ———————|———————        MINISATELLITE
    GACACTCAGTCACCCA 5'
```

pMS1.1b  3'flanking sequence, starting at the Eco Rv site about 50bp
from the end of minisatellite

EcoRV
  |
5' TGGATATCTCATGCATGGGAGCCCAGATTGGGTGGTCCTGGCCCTCATGGGTTGCATATG
   ————|—————————|—————————|—————————|—————————|—————————|—————————|.
3' ACCTATAGAGTACGTACCCTCGGGTCTAACCCACCAGGACCGGGAGTACCCAACGTATAC

   CTTCTTTGCTGCCTGTGCTGTAAGAACCATGCTAAGAACAGACCTGTGCCCCTGGGCTCT
   ————————|—————————|—————————|—————————|—————————|—————————|
   GAAGAAACTACGGACACGACATTCTTGGTACGATTCTTGTCTGGACACGGGGACCCGAGA

   CAGCAGTGCATGTGCCAGGGAGCTGTGGGTGTGGAAGCAGCAGGAAAAACAGCTGTGCCA
   ————————|—————————|—————————|—————————|—————————|—————————|
   GTCGTCACGTACACGGTCCCTCGACACCCACACCTTCGTCGTCCTTTTTGTCGACACGGT

   GGTCGGGGAGGGGAAGGGACAGAGAACCAGGGGCAGGGAAAGAGTTCCAAGCTCCTTGGC
   ————————|—————————|—————————|—————————|—————————|—————————|
   CCAGCCCCTCCCCTTCCCTGTCTCTTGGTCCCCGTCCCTTTCTCAAGGTTCGAGGAACCG

   CACCGNACCATGTGCTCATGGCCTCCTCCCCGGCTGTCAGTTTCCCTATCTGTAAAATGA  3'
   ————————|—————————|—————————|—————————|—————————|—————————|
   GTGGCNTGGTACACGAGTACCGGAGGAGGGGCCGACAGTCAAAGGGATAGACATTTTACT  5'

EP 0 370 719 A2

pMS1 - the tandem repeat block is abbreviated to the two outermost repeats (in capitals) separated by X's, a novel repeat is underlined.

```
   1   gatcctaaccctccaaccatgaagcccaaatctgaccccatgacccctgatttgtaaaccccgcctggcc
  73   tgtacaggtccagccccagctgctgtgccttccccggttcagtctcctagtgaaggtgcgggagctgagca
 145   atgaaggcactgttggggaaactgtagcagggaaggggcaggctctatggagggtccttgatacacaccgc
 217   acccccaagccttaagcagacatcaggaaaacccagtgaagggccgcaggagagatgtgtggacctgcacc
 289   tgcacctccgggctctgtccatgccccaccagctacgcacttgtaccagttggtgagcgtcatcatgacgt
 361   cagtgaggccagcaccaggcagaagtggaagaaggagtagctgtaggtgacgccgtcctgctcgttgtcaa
 433   ggcccggccctcacaggctgccacctgctgctgctgctgtgtggcgtctagcataggtgggcactcct
 505   ggtctgcatcaggctgttcacctgccggtggtctgaggagcgcagactgcaggggagggaggaaggtgggc
 577   aatagatagctctggcgttggtcataacactggacacaaatggtcccagagcctcagaggccagcgacccg
 649   ttactgggcctgggagttaacaaactgctttgcctctcaactctacccctaccccactcccattttctgt
 721   tgaccttgagcaaaacacttaacctctctgagcctctgattccttgtctgtaaaataagggataatcgtac
 793   tcctctagagggctgctggggggactagacggaataatgatgtaaggtctctagcatagtgcttggcacag
 865   atataaattcaatcaacagtcactattgttaggattgttcctttgccatttccataaacacgtatcgagta
 937   ctaatactacaaagtaccataccaggtactacatccattaagaatgttaataattaatcacgctcatttgc
1009   attgattttaagtttccattttattagtgtcaggtggtttttaccaatggcctcccttttccttcatgcttac
1081   ctaaaaaaagaacaacagtaatgattgtatgtcatgcttttctgtgatgagccttgatgttttttaacagaa
1153   ttgatagtttgagacataaaaattttttgaaaaaccaactcacgtttccaaacaaaagtgaaaaggagagct
1225   gattccagccccgccacccagcaaattgagaaatcaccccttgctgtgagtcagtgggtCCCTATCCAxx
1297   xxxxxxxxxxxxCTATCTACCaaataccatatggatatctcatgcatgggagcccagattgggtggtcctg
1369   ccctcatgggttgcatatgcttctttgctgcctgtgctgtaagaaccatgctaagaacagacctgtgcccc
1441   gggctctcagcagtgcatgtgccagggagctgtgggtgtggaagcagcaggaaaaacagctgtgccaggtc
1513   gggagggaagggacagagaaccaggggcagggaaagagttccaagctccttggccaccggcaccatgtgc
1585   catggcctcctccccggctgtcagtttccctatctgtaaaatgaaggtaatcaggccaggagtggtgcact
1657   ctgtaatccaaacactttggcaggctgaggtaggaggattgcttgggccaggagttcaaggccatcctggg
1729   aacatagtatctctacaaaaaaaa
```

The minisatellite repeat sequence in clone λMS29. The region in the 39bp consensus repeat sequence similar to the 3' end of the minisatellite core sequence (GGAGGTGGGCAGGARG, Jeffreys et al,. 1985 ) is marked with asterisks. Deviations from the consensus sequence are shown for two blocks of 4 contiguous repeat units (a-d, e-h) cloned at random from λMS29. The 39bp repeat sequence consists of 13 diverged repeats of PyAG.

```
                         *********
consensus    TTGCAGTAGCTGTGGCAGGAGGAGTAGCAGCATCAGCAG = (YAG)13
             ─→─→─→─→─→─→─→─→─→─→─→─→─→
```

```
a                            -C  G                          T
b                                                        G
c              G                                         G
d              G                          A
```

```
e         C                                        G
f                               ---                 G
g                               ---              G  G      C
h         C                     ---              G
```

### pMS31 5'flanking sequence extending into minisatellite

```
5' GATCCACTCGGAACCACCTGCAGTTAGGAGCAAGCCTAGAATGTTCTGGAAGGATTGAAG
   ──────|──────|──────|─────|──────|──────|
3' CTAGGTGAGCCTTGGTGGACGTCAATCCTCGTTCGGATCTTACAAGACCTTCCTAACTTC

   CCAGCCTTGTCTGAGGCCCTGGGAAAGTGGCCTGGACATGGGGATGTGGCTGGAGGACCC
   ──────|──────|──────|─────|──────|──────|
   GGTCGGAACAGACTCCGGGACCCTTTCACCGGACCTGTACCCCTACACCGACCTCCTGGG

   GAGGAAGATGCTGAAGTCCTGTGTGAGGCCCGGTCTGGGAGCCACGGCCCCTCCCCCACT
   ──────|──────|──────|─────|──────|──────|
   CTCCTTCTACGACTTCAGGACACACTCCGGGCCAGACCCTCGGTGCCGGGGAGGGGGTGA

   CAGTCCGGCCTGCTGGGGTTTCCTGCCGGGCCTCCCTCAGAGCCCACGGCTCCCCAGGTG
   ──────|──────|──────|─────|──────|──────|
   GTCAGGCCGGACGACCCCAAAGGACGGCCCGGAGGGAGTCTCGGGTGCCGAGGGGTCCAC

   GCTCTGGCCCGGGAGCCACAGGCACAACCTAGGCAGGGGAAGCCGCATGCACAATGTTGG
   ──────|──────|──────|─────|──────|──────|
   CGAGACCGGGCCCTCGGTGTCCGTGTTGGATCCGTCCCCTTCGGCGTACGTGTTACAACC

   CTCTTCCCTTTGCACGCTGGACGGTGGCGTTTTGCCTTTCGCCTTGGGCTCAGGAGGGGC
   ──────|──────|──────|─────|──────|──────|
   GAGAAGGGAAACGTGCGACCTGCCACCGCAAAACGGAAAGCGGAACCCGAGTCCTCCCCG

   TGGGGGGTCAGGAGGGGGCCATGAAGGGGACCTGGCCTTGG 3'
   ──────|──────|──────|─────|         MINISATELLITE
   ACCCCCCAGTCCTCCCCGGTACTTCCCCTGGACCGGAACC 5'
```

EP 0 370 719 A2

pMS31 - the tandem repeat 31B is shown in full capitals.

```
  1 gatccactcggaaccacctgcagttaggagcaagcctagaatgttctggaaggattgaagccagccttgtc
 73 gaggccctgggaaagtggcctggacatggggatgtggctggaggacccgaggaagatgctgaagtcctgtg
145 gaggcccggtctgggagccacggcccctcccccactcagtccggcctgctggggtttcctgccgggcctcc
217 tcagagcccacggctccccaggtggctctggcccgggagccacaggcacaacctaggcaggggaagccgca
289 gcacaatgttggctcttccctttgcacgctggacggtggcgttttgcctttcgccttgggctcaggagggg
361 tggggggtcaggaggggccatgaaggggacctggccttggCTGTCCACCTCCCACAGACAxxxxxxxxxxxx
433 xxxTCTACCTCCCACAGACACTGCCggccggatgggcGTGTGGGGACGGTGTGCCGGTGTGGGGACGGGGT
505 CAGGTGTGGGGACGGGGTGCAGGTGTGGGGACGGGGTGCAGGTGTGGGGACGGCGTGCAGGTGTGGGGACG
577 GGTGCAGGTGTGGGGACGGCGTGCTGTGGGGATC   610
```

FLANKING SEQUENCE 3' TO THE MS31 LOCUS

MINISATELLITE

```
MINISATELLITE  5'  CCGGCCGG ATGGGCGTGT GGGGACGGTG TGCCGGTGTG GGGACGGGGT
               3'  GGCCGGCC TACCCGCACA CCCCTGCCAC ACGGCCACAC CCCTGCCCCA


                   GCAGGTGTGG GGACGGGGTG CAGGTGTGGG GACGGGGTGC AGGTGTGGGG ACGGCGTGCA
                   CGTCCACACC CCTGCCCCAC GTCCACACCC CTGCCCCACG TCCACACCCC TGCCGCACGT


                   GGTGTGGGGA CGGGGTGCAG GTGTGGGGAC GGCGTGCTGT GGGGATC 3'
                   CCACACCCCT GCCCCACGTC CACACCCCTG CCGCACGACA CCCCTAG 5'
```

5'flanking sequence of pMS32.6 extending into minisatellite

```
5'  GATCACCGGTGAATTCCACAGACACTTAAAAGCAAAAATAATAATTTGTTGAATACAGTG
    ———————I————————I————————I————————I————————I————————I
3'  CTAGTGGCCACTTAAGGTGTCTGTGAATTTTCGTTTTTATTATTAAACAACTTATGTCAC


    AGTTCTAAATTTCTCTTCAAAGAATCAGTATGTCAGTATGTTCAGTTCTTTGTTCTCCAT
    ———————I————————I————————I————————I————————I————————I
    TCAAGATTTAAAGAGAAGTTTCTTAGTCATACAGTCATAGGAGTCAAGAAAGCCGAGGTA


    TTTAAAGTTGAACTTCCTCGTTCTCCTCAGCCCTAGTTTCACTAAACAACCTTTTCCAAC   3'
    ———————I————————I————————I————————I————————I————————I
    AAATTTCAACTTGAAGGAGCAAGAGGAGTCGGGATCAAAGTGATTTGTTGGAAAAGGTTG   5'
```

MINISATELLITE

pMS32.6  3'flanking sequence extending from minisatellite

```
                                              5'   AACCACCCTTCCCAC
                    MINISATELLITE            ————————I————————I
                                              3'   TTGGTGGGAAGGGTG


CAAACTACTCACCCCGCCACTCTGGCTCATACCCCTGCTCTCTTTAAAGTAGCCAATCGG
————————I————————I————————I————————I————————I————————I
GTTTGATGAGTGGGGCGGTGAGACCGAGTATGGGGACGAGAGAAATTTCATCGGTTAGCC


AATTAGCTTAGACTGTGCAGTCCAACCCTAGCCGATACGGGAACGACGCATCAGTAGGGG
————————I————————I————————I————————I————————I————————I
TTAATCGAATCTGACACGTCAGGTTGGGATCGGCTATGCCCTTGCTGCGTAGTCATCCCC


CTACCTGTGTCAGGAATCAGAACCCCTTCCCCTCCCTTGTTCAGGTGTGCTCTGGCCATT
————————I————————I————————I————————I————————I————————I
GATGGACACAGTCCTTAGTCTTGGGGAAGGGGAGGGAACAAGTCCACACGAGACCGGTAA


GCTCCATCTGCGAGTCGCACCCTTCTAGAGAAGTAAAATTGCCTTGCTGAGAAAATTAAA
————————I————————I————————I————————I————————I————————I
CGAGGTAGACGCTCAGCGTGGGAAGATCTCTTCATTTTAACGGAACGACTCTTTTAATTT


CTTATGTTTGAGTGGTATTTCTTTTGCAGCACCAAAAATTTATTTACAACAAATTCTACA   3'
————————I————————I————————I————————I————————I————————I
GAATACAAACTCACCATAAAGAAAACGTCGTGGTTTTTAAATAAATGTTGTTTAAGATGT   5'
```

EP 0 370 719 A2

PMS32 - retrovital LTR underlined L1 sequence in bold

```
    gatcaccggtgaattccacagacacttaaaagcaaaaataataatttgttgaatacagtgagttctaaatt
73  ctcttcaaagaatcagtatgtcagtatgttcagttctttgttctccatttttaaagttgaacttcctcgttc
145 cctcagccctagtttcactaaacaaccttttccaacagttctaatcagtagttaacgtctattCCCCTGGT
217 ACCTGCTCCATTCTGAGTCAxxxxxxxxxxxxxxxxxACCTGAATCATCCTGAGTCACCTGTTCTGTaaccac
289 cttcccaccaaactactcaccccgccactctggctcataccctgctctctttaaagtagccaatcggaat
361 agcttagactgtgcagtccaaccctagccgatacgggaacgacgcatcagtagggggctacctgtgtcagga
433 tcagaacccttcccctcccttgttcaggtgtgctctggccattgctccatctgcgagtcgcacccttcta
505 agaagtaaaattgccttgctgagaaaattaaacttatgtttgagtggtatttcttttgcagcaccaaaaat
577 tatttacaacaaattctacacaaattttccagaaactggaaatggagagcttatgttacccaattttaag
649 tttactagaaagatacaatagacgtgacagggtggtattggtagaagaatagacacgaagatc
```

## Flanking sequences of 33.1

```
5' TGTACACTCC CAGTGCCCGA GAGGATGGGG TTGAGAGCTA AGCTGAGAAA GTCTATCTCT
3' ACATGTGAGG GTCACGGGCT CTCCTACCCC AACTCTCGAT TCGACTCTTT CAGATAGAGA

   GAATTTCANT GCTGATAAAA ACAACCGAGC CTGCCGGGGC AGGGGCTTGC TTTCTCCACG
   CTTAAAGTNA CGACTATTTT TGTTGGCTCG GACGGCCCCG TCCCCGAACG AAAGAGGTGC

   GATGGGATGC CACAACTGC
   CTACCCTACG GTGTTGACG
```

## MINISATELLITE

```
                     TTTCCTGAA CATCGTACCC CCCACCCCCC AGAAGCTTGT
                     AAAGGACTT GTAGCATGGG GGGTGGGGGG TCTTCGAACA

   GGGTGACACG GCACGGTGGC TTAGGGACAC AGATGATAGA AGCTGGCTGC ATAAAAGACA
   CCCACTGTGC CGTGCCACCG AATCCCTGTG TCTACTATCT TCGACCGACG TATTTTCTGT

   AGTCTTTACA GAGTCTTGTC AAGCCCTCAG GTCTGCGCAC GTCTGATCTA ACCAAAGTTT
   TCAGAAATGT CTCAGAACAG TTCGGGAGTC CAGACGCGTG CAGACTAGAT TGGTTTCAAA

   TTCCTGTGGA 3'
   AAGGACACCT 5'
```

### Flanking sequences of 33.4

```
5' ATCAAGGAGG CAGTGAGAGA TGAACTGGAA GTGACCAGGG GCTGCCAGCA AACCCCCTCC
3' TAGTTCCTCC GTCACTCTCT ACTTGACCTT CACTGGTCCC CGACGGTCGT TTGGGGGAGG

   ACCGAGAAGA TGACTTTCAC CTACTATACA GCAGAAAACC AAAAGCCAAG ATAAAAATCG
   TGGCTCTTCT ACTGAAAGTG GATGATATGT CGTCTTTTGG TTTTCGGTTC TATTTTTAGC

   CTGGGGAGGG GCAGGGATGG GGACCGGGC CAGACCCCAG CTGCTGAGCA CGCGCCACCT
   GACCCCTCCC CGTCCCTACC CCCTGGCCCG GTCTGGGGTC GACGACTCGT GCGCGGTGGA
```

### MINISATELLITE

```
   CCTAAGCAGG TTCTGGTGGC CTCCTGGCTG GGTGTAGGCA GAGGCTGGCT CCCGGAGGCC
   GGATTCGTCC AAGACCACCG GAGGACCGAC CCACATCCGT CTCCGACCGA GGGCCTCCGG

   GCAGGGGGGC TCTGAGAAGG GGGCGGCCTG AGGGGGAGCC CAGGACAGCC CCTATGCTGC
   CGTCCCCCCG AGACTCTTCC CCCGCCGGAC TCCCCCTCGG GTCCTGTCGG GGATACGACG

   CCCCGTCCAG CCCGGCCCCT CAGGCTGTGT TTCCTGAGAC CTCGCTAGTC TAACCCAAAC
   GGGGCAGGTC GGGCCGGGGA GTCCGACACA AAGGACTCTG GAGCGATCAG ATTGGGTTTG

   CTCCCAGCTC TGTCATTCAA AGGGATGGAT TAGGTCCCCG TGGGTCCATT TCCCT 3'
   GAGGGTCGAG ACAGTAAGTT TCCCTACCTA ATCCAGGGGC ACCCAGGTAA AGGGA 5'
```

### Flanking sequences of 33.6

```
5' TGTGAGTAGA GGAGACCTCA CATTTGACCT TGGAAAGT
3' ACACTCATCT CCTCTGGAGT GTAAACTGGA ACCTTTCA
```

### MINISATELLITE

```
              GGTTG CTCCTCACTC TGTGGTCTTT GTCTGTCCAG ACCTTCCCTT
              CCAAC GAGGAGTGAG ACACCAGAAA CAGACAGGTC TGGAAGGGAA

   CTTGGATTGA GTAATGTCTC ACCTGCTTGC 3'
   GAACCTAACT CATTACAGAG TGGACGAACG 5'
```

21

pMS43 - <u>Alu</u> sequences are in bold, and short tandem repeats are underlined. The minisatellite arrays are abreviated to the two outermost repeats (in capitals) separated by X's, sequencing 3' of 43A resumes at a <u>Sma</u> I site within the last repeat unit, and so only these 6 bases are shown in capitals.

```
   1    gatctatacatgtTTACACACATGCCACACACCCTTCCCAAGGCCGTCCCTATACCCAxxxxxxxxxxxxxx
  73    xCCCGGGggccatcggcgcagtggcctcagcgtccagcctccccgtcctccggagctctctctggtggggg
 145    cccagtcctcctcgtcttccggagctctctctctctggtgggggcccccacctggccgacctgaaggtcagg
 217    catccactctttggggaaacgaagttgggttcgtaggggcagctctggtgttgttggtccttccctccaga
 289    accacgtctcttcgtctggctcccaggagacocccagttctatttctatttctcccgcctctgtcatcccct
 361    aaactgcttctgcatagcccccactctgcctctcaggctttaaccccaactgtgagccccacctggctgtc
 433    tggggccactgaactccacgtgtccatcgtggctctccccaacatgccaccacgcaccggctggcattctg
 505    ccctatgaagccctgcctgcctggggactgggaatcagccgctgtcccagcccacccgtcaatcacccatc
 577    atcaccggccaatcacccgtcaatcaccgtctcttgccagctttgcatcctaaatattttttggaagagtc
 649    cgtgtccgccacacccccacaagaaagaaagttcaccctactgggctcaacagtaaagatgttaatgaaggg
 721    agcttagggatgtgtggttaggggcatagggacagacggggggcagtgcaacctcagggggcggcaacgtgca
 793    ttctcgacaccggcagagtgaggcctcgggcgccactcggaccacggtgcagcttccaacaacctgtggtg
 865    ctccacgggccaagacagcctgcgtgcctccacgggccaagagcacaaggtgggaggagggaaggagctgg
 937    ggactggaaagcggtggtcagcatctgagggcccggcacgTGGGGCTAGACGGGGGATGTGGTGAxxxxxxx
1009    xxxxxxxxCGGGGGCCTGGTGATGGGGacaggtgagtctcctgtccctcaccaggagaactgggttctggt
1081    ctcagcctgtctcccaccgtagagcaacctctgaccgccagacaggacaccaaggtgaaaactttcaagcc
1153    ccaggacgggagagaggtcaggggggngaggtcggggnngaggtggcagggagggcagggaggtcagggagt
1225    gagtaaacgctggcgcggccacagctgtgccttgtcaggggatgaaggtgaaagacagaacccgcggtgac
1297    caggctcccctgccccgcggaggagtcaggccatcagaaaggcccctatgtcagccaggcgtggtggttca
1369    acctggaatcccagcactgtgggaggcagaggcgggcagatc
```

### FLANKING SEQUENCES OF THE MS51 LOCUS

```
5' GATCAGCGAA CTTCCTCTCG GCTCCCGATA TCCTCCTCGA TACGCACTCT GCCACAACGG
3' CTAGTCGCTT GAAGGAGAGC CGAGGGCTAT AGGAGGAGCT ATGCGTGAGA CGGTGTTGCC

   GCAGGGTCCC TTTCAGCGTC TCATCCACAG TGAACGGGAG TTGAGGCTTT CTTAGCGGAG
   CGTCCCAGGG AAAGTCGCAG AGTAGGTGTC ACTTGCCCTC AACTCCGAAA GAATCGCCTC

   GGGCTGGAGG GAC
   CCCGACCTCC CTG
```

### MINISATELLITE

```
            CCTC CCTGCGGTTT CCGGATGCTA CGGGGTGGAT CGGAGTGTGG
            GGAG GGACGCCAAA GGCCTACGAT GCCCCACCTA GCCTCACACC

   TGTTAAGCAC ATCTGGACAC GCTCTGTCCG AGACACATAG TCCCCAGGCG ACCTACAGCC
   ACAATTCGTG TAGACCTGTG CGAGACAGGC TCTGTGTATC AGGGGTCCGC TGGATGTCGG

   ACAGCCTGAC CTCCTGAAAA TTTCCCAGCT TCCCACAGTC CTCAATGTGG AAACCAGTGC
   TGTCGGACTG GAGGACTTTT AAAGGGTCGA AGGGTGTCAG GAGTTACACC TTTGGTCACG

   CCAAAGGCCA CCTGCCCACA CTGGCACCGA ATTC 3'
   GGTTTCCGGT GGACGGGTGT GACCGTGGCT TAAG 5'
```

### MS51- Sequence across the adjacent Hinf I site

```
  1  AGTGGATTTT CCCACTTCCC TCTGTTGTCT ATAATGAATG ACCACTACAT

 51  TTTTAACCCC AAAAGCTTCT CATATGAAGA GGAGACTTCT TTCAGCAGCA

101  GGCAGGGCAC CTTCTCTGAA GCTCGCCAGC AAGCACCAAC CGACTCCCTG

151  GCCACGGCAG CTGACGCCCA GGCAGTGACC CCACACGGGA GGGCTCTCGA

201  AAAGRCTGGA GCTCCCATGA C
```

EP 0 370 719 A2

pMS228 - sequnce from a 2kb subclone of DNA surrounding 228B.  Alu sequences are
in bold, and short tandem repeats are underlined.  The minisatellite arrays are
abbreviated to the two outermost repeats (in capitals) separated by X's.

```
aggccacccccaccaacaacataaccagagggggaaggaagtcaggcccctttctcactctgagaagctggtg
ctgggatttttaggctgtcacgacgattccacccggccagggcaggcccgaaccggccggaggccacaggag
accaatgagcctggctggactcctgcaaaccttgagggacgccgagattcacattcactgaattttcatgc
acgtgacactcttcttcttttgttccctcccgccctgccccacggagccatttacaaacataaaaccattt
taaaccatttttaaatggtttaaaacctgtttctgtaccaagtggtacagaaataggggccagcccccggt
GAGAGGGGGACAGGGtcatcagggtgcttagggtgggctccggggcgtctgcaccaccaggcgcacagccc
ggaggtggcaggagtcatccgttctgaaacagccagaggtacaacctcgtcgtccaggcaccggccgagtt
actgcggaggccccacaggaacaatccgggagggtggggtggtgcctgcctggcagctgcggggggctgggt
gggaagggctactccaccctggaggcccagctcacaccaacctcctagcccctgacgtcccaccaggcagc
tcacaaggttacaggtcggttccttctccactggattcctcccacatcgggtgacctgaccacacacacgg
aggtgcccagcggtggtcccagccccaacatctcaagagcaggacacccgagtggagatactaggtcacag
aatgtctccacacagacattcaggcaggttcgagggaagaagacagcttcccggccactctcccaccacgc
acacccggtgggctcctctcctcaacctgggcccacattctctcccaggttactcacatcactcagtcatc
ctcacatcactcacatcgccccatgacatccgcctctgagctgccagcctccctcccccagcccctttcttc
ttccctccctccctccctccttttcttttttttgaaacaggtcacccaggctggagttcagtggcacaatct
gtagttggaaacgtaactgggcaccaccatgcccagctattttttttttttttttcagtagagatgaggtctc
ctacattacccaggctggtctcagactcctggtctcaagcaatcttctcaccttggcctcccaaagtgcta
gattacaggtgtgagccactgcgcccgacttccccagccccttttctgacccacagcctgggatc
```

Flanking sequences of the hypervariable region 5'
to the human insulin gene
(AM. J. HUM. GENET., 1986, 39, 291–229.)


5' CTGGGGCTGC TGTCCTAAGG CAGGGTGGGA ACTAGGCAGC CAGCAGGGAG GGGACCCCTC
3' GACCCCGACG ACAGGATTCC GTCCCACCCT TGATCCGTCG GTCGTCCCTC CCCTGGGGAG

CCTCACTCCC ACTCTCCCAC CCCCACCACC TTGGCCCATC CATGGCGGCA TCTTGGGCCA
GGAGTGAGGG TGAGAGGGTG GGGGTGGTGG AACCGGGTAG GTACCGCCGT AGAACCCGGT

TCCGGGACTG GGG          MINISATELLITE                         ACAG
AGGCCCTGAC CCC                                                TGTC

CAGCGCAAAG AGCCCCGCCC TGCAGCCTCC AGCTCTCCTG GTCTAATGTG GAAAGTGGCC
GTCGCGTTTC TCGGGGCGGG ACGTCGGAGG TCGAGAGGAC CAGATTACAC CTTTCACCGG

CAGGTGAGGG CTTTGCTCTC CTGGAGACAT TTGCCCCCAG 3'
GTCCACTCCC GAAACGAGAG GACCTCTGTA AACGGGGGTC 5'


FLANKING SEQUENCES OF 5' ALPHA GLOBIN HVR
(AM. J. HUM. GENET., 1988, 43, 249–256)



5' TTAGATGCTC GCCG   MINISATELLITE   CTGTGCGGG AAGCCCGAAA TCCTTA 3'
3' AATCTACGAG CGGC                   GACACGCCC TTCGGGCTTT AGGAAT 5'



FLANKING SEQUENCES OF THE ALPHA GLOBIN 3' HVR
(EMBO, 1986, 5, 1957–1863)



5' AGTCCCACCT GCAGGAAAAG GTGCAGGTAA GG
3' TCAGGGTGGA CGTCCTTTTC CACGTCCATT CC

MINISATELLITE

AGGAACAGCA ACACGCAGGG AATATCGACA TGGGTGATGC
TCCTTGTCGT TGTGCGTCCC TTATAGCTGT ACCCACTACG

CTGCAAAGCA CAGCATCAAT CCCAAGTGAC TGA 3'
GACGTTTCGT GTCGTAGTTA GGGTTCACTG ACT 3'


25

Flanking sequences of hypervariable region at the Zeta globin locus
(PNAS, 1983, 80, 5022–5026)

```
5' ACACCCATCA ATGGGAGCAC CAGGACAGACAT GGAGGCTAAT GTCATGTTGT AGACAGGAT
3' TGTGGGTAGT TACCCTCGTG GTCCTGTCTGTA CCTCCGATTA CAGTACAACA TCTGTCCTA

   GGTGCTGAGC TGACCACACC CACATTATTA GAAAATAACA GCACAGGCTT GGGGTGGAGG
   CCACGACTCG ACTGGTGTGG GTGTAATAAT CTTTTATTGT CGTGTCCGAA CCCCACCTCC

   CGGGACACAA GACTAGCCAG AAGGAGAAAG AAAGGTGAAA AGCTGTTGGT GCAAGGAAGC
   GCCCTGTGTT CTGATCGGTC TTCCTCTTTC TTTCCACTTT CGACAACCA CGTTCCTTCG

   TCTTGGTATT TTCAACGGCT              MINISATELLITE              AGC
   AGAACCATAA AAGTTGCCGA                                         TCG

   TACAGGGAGA AAAGACTTGG TGCTGTGGGC CTGCCTTGGG GCTGGTGGTA CAGCCCTTAT
   ATGTCCCTCT TTTCTGAACC ACGACACCCG GACGGAACCC CGACCACCAT GTCGGGAATA

   CTGCTGCCCT CAGGATCTCC CGGCCCCTCT CGTCCAGGCC CCTGCAACCC CATGCCCCAG
   GACGACGGGA GTCCTAGAGG GCCGGGGAGA GCAGGTCCGG GGACGTTGGG GTACGGGGTC

   CCTCTGAGGA CCAAAGGCGC CCCTGCTTGG GAAGAGGGGG CTCAGGGGAG TCGCCTGACC
   GGAGACTCCT GGTTTCCGCG GGGACGAACC CTTCTCCCCC GAGTCCCCTC AGCGGACTGG

   CGGTTCCAAG CCAGGCTGAT TTACCGTTGT TAACATCCTA GTGCACGCAT CCCTCTGCCT
   GCCAAGGTTC GGTCCGACTA AATGGCAACA ATTGTAGGAT CACGTGCGTA GGGAGACGGA

   CATGCACCCA ACTCCAAGGC CTGGTACAC 3'
   GTACGTGGGT TGAGGTTCCG GACCATGTG 5'
```

## FLANKING SEQUENCES OF HA RAS LOCUS
### (NATURE, 302, 33-37)

```
5' TGCTCCAAGG GGCTTGCCCT GCCTTGGGCC AAGTTCTAGG TCTGGCCACA GCCACAGACA
3' ACGAGGTTCC CCGAACGGGA CGGAACCCGG TTCAAGATCC AGACCGGTGT CGGTGTCTGT

   GCTCAGTCCC CTGTGTGGTC ATCCTGGCTT CTGCTGGGGG CCCACAGCGC CCCTGGTGCC
   CGAGTCAGGG GACACACCAG TAGGACCGAA GACGACCCCC GGGTGTCGCG GGGACCACGG

   CCTCCCCTCC CAGGGCCCGG GTTGAGGCTG GGCCAGGCCT CTGGGACGGG GACTTGTGCC
   GGAGGGGAGG GTCCCGGGCC CAACTCCGAC CCGGTCCGGA GACCCTGCCC CTGAACACGG

   CTGTCAGGGT TCCCTATCCC TGAGGTTGGG GGAGAGCTAG CAGGGCATGC CGCTGGCTGG
   GACAGTCCCA AGGGATAGGG ACTCCAACCC CCTCTCGATC GTCCCGTACG GCGACCGACC

   CCAGGGCTGC AGGGAC              MINISATELLITE
   GGTCCCGACG TCCCTG

   GAGTGACCAG CTTCCCCATC GATAGACTTC CCGAGGCCAG GAGCCCTCTA GGGCTGCCGG
   CTCACTGGTC GAAGGGGTAG CTATCTGAAG GGCTCCGGTC CTCGGGAGAT CCCGACGGCC

   GTGCCACCCT GGCTCCTTCC ACACCGTGCT GGTCACTGCC TGCTGGGGGC GTCAGATGCA
   CACGGTGGGA CCGAGGAAGG TGTGGCACGA CCAGTGACGG ACGACCCCCG CAGTCTACGT

   GGTGACCCTG TGC 3'
   CCACTGGGAC ACG 5'
```

| Panel of Single Locus Probes Proposed by Ray White | | | | | | | | | | |
|---|---|---|---|---|---|---|---|---|---|---|
| | | | | Number of Alleles | Size Range | Range of Allele Frequencies | Probability of 2 Individuals Matching | Prob. of 2 Sibs Matching | Exclusion* | |
| D17530 | YNZ22 | Zeta globin | Hinfl | 14 | 0.5-1.3kb | 0.0033-0.29 | 0.0377 | 0.33 | 70% | NAR 16,5705 |
| D2544 | YNH24 | HBV-2 | Hinfl | >30 | 1-5kb | 0.033-0.05 | 0.001 | 0.26 | 95% | NAR 15,10073 |
| D957 | pEFD126.3 | HBV-4/5 | Hinfl | 5 | 1-2kb | | 0.0349 | 0.33 | 71% | NAR 15,10607 |
| D14513 | MLJ14 | Myoglobin | Hinfl | >20 | 4-15kb | 0.0046-0.096 | 0.0042 | 0.27 | 91% | NAR 16,381 |
| D19520 | JCZ3.1 | Zeta | Hinfl | >10 | 1.5-4kb | | 0.0554 | 0.35 | 65% | NAR 16,1229 |
| D16583 | EKMA21 | | Alu | | | | 0.0302 | 0.32 | 74% | |
| D1574 | CYNA13 | | Alu | | | | 0.0065 | 0.28 | 88% | |
| | pEFD64.1 | HBV4/5 | Hinfl | | | | 0.091 | 0.396 | 55% | |
| | | | | | | TOTAL | $6\times10^{-15}$ | $1\times10^{-4}$ | | |

* A priori probability that a falsely accused putative father will be excluded by probe

| Additional Single Locus Probes Cloned by Ray White | | | | | | | |
|---|---|---|---|---|---|---|---|
| | | | | | Heterozygosity | | |
| D1754 | pCMM86 | Myoglobin | HinfI | >10 | 5-1.3kb | 90% | NAR 16,5223 |
| D17526 | EFD52 | HBV-4 | MspI* | >8 | 5-10kb | 83% | NAR 16,786 |
| D17528 | pYNH37.3 | HBV-2 | MspI* | 5 | · 2-4kb | 78% | NAR 16,782 |
| D75396 | pJCZ67 | Zeta globin | MspI* | 4 | 3-6kb | 80% | NAR 16,4191 |
| D13552 | cMHZ47 | YNZ22 | MspI* | >10 | 1.5-3.0kb | 80% | NAR 16,3119 |
| D14523 | CKKA39 | | MspI* | >10 | 2-4.5kb | 83% | NAR 16,3120 |

* Use of HinfI not investigated

REFERENCES

1. Bell G I, Selby J M and Rutter W J
(1982) Nature 295, 31-35.
2. Capon D J, Chen E Y, Levinson A D, Seeburg PH and Goeddel D V (1983) Nature 302, 33-37.
3. Goodbourn S E Y, Higgs D R, Clegg J B and Weatherall D J
(1983) Proc. Nat. Acad. Sci. USA 80, 5022-5026.
4. Jeffreys A J
(1987) Biochem. Soc. Trans. 15, 309-317
5. Jeffreys A J, Wilson V and Thein S L
(1985) Nature 314, 67-73
6. Jeffreys A J, Wilson V and Thein S L
(1985) Nature 316, 76-79
7. Vassart G, Georges M, Monsieur R, Brocas H, Lequarre A S and Christophe D
(1987) Science 235, 683-684
8. Wong Z, Wilson V, Jeffreys A J and Thien S L
(1986) Nucleic Acids Res. 14, 4605-4616
9. Nakamura Y, Leppert M, O'Connell P, Wolff R, Holm T, Culver M, Martin C, Fujimoto E, Hoff M, Kumlin E and White R
(1987) Science 235, 1616-1622.
10. Wong Z, Wilson V, Patel I, Povey S and Jeffreys A J
(1987) Ann. Hum. Genet. 51, 269-288.
11. Reeder S T, Breuning M H, Davies K E, Nicholls R D, Jarman A P, Higgs D R, Pearson P L and Weatherall D J
(1985) Nature 317, 542-544.
12. Donis-Keller H, Green P, Helms C, Cartinhour S, Weiffenbach B, Stephens K, Keith T P, Bowden D W, Smith D R, Lander E S, Botstein D, Akots G, Rediker K S, Gravius T, Brown V A, Rising M B, Parker C, Bricker A, Phipps P, Muller-Kahle H, Fulton T R, Ng S, Schumm J W, Barker D F, Crooks S M, Lincoln S E, Daly M J and Abrahamson J
(1987) Cell 51, 319-337.
13. Nakamura Y, Lathrop M, O'Connell P, Leppert M, Barker D, Wright E, Skolnick M, Kondoleon S, Litt M, Lalouel J -M and White R
(1988) Genomics 2, 302-309.
14. Jeffreys A J, Brookfield J F Y and Semeonoff R
(1985) Nature 317, 818-819.
15. Baird M, Balazs I, Giusti A, Miyazaki L, Nicholas L, Wexler K, Kanter E, Glassberg J, Allen F, Rubinstein P and Sussman L
(1986) Am.J.Hum.Genet 39, 489-501.
16. Thein S L, Jeffreys A J and Blacklock H A
(1986) Lancet ii, 37.
17. Knowlton R G, Brown V A, Braman J C, Barker C, Schumm J W, Murray C, Takvorian T, Ritz J and Donnis-Keller H
(1986) Blood 68, 378-385.
18. Gill P, Jeffreys A J and Werrett D J
(1985) Nature 318, 577-579.
19. Gill P, Lygo J E, Fowler S J and Werrett D J
(1987) Electrophoresis 8, 38-44.
20. Mullis K, Faloona F, Scharf S, Siki R, Horn G and Erlich H
(1986) Cold Spring Harbor Symp. Quant.Biol. 51, 263-273.
21. Saiki R K, Gelfand D H, Stoffel S, Scharf S J, Higuchi R, Horn G T, Mullis K B and Erlich H A
(1988) Science 239, 487-491.
22. Higuchi R, Von Beroldingen C H, Sensabaugh G F and Erlich H A
(1988) Nature 332, 543-546.
23. Li H, Gyllensten U B, Cui X, Saiki R K, Erlich H and Arnheim N
(1988) Nature, 335, 414-417.

24. Wong C, Dowling C E, Saiki R K, Higuchi R G, Erlich H A and Kazazian H H
(1987) Nature 330, 384-386.

25. Saiki R D, Scharf S J, Faloona F, Mullis K B, Horn G T, Erlich H A and Arnheim N
(1985) Science 230, 1350-1354.

26. Jeffreys A J and Morton D B
(1987) Animal Genetics 18, 1-15.

27. Vieira J and Messing J
(1982) Gene 19, 259-268.

28. Feinberg A P and Vogelstein B
(1984) Anal.Biochem. 137, 266-267.

29. Maniatis T, Fritsch E F and Sambrook J
(1982) Molecular cloning: a laboratory manual (Cold Spring Harbor Laboratory, N.Y.).

30. Armour J A L, Patel I, Thein S L, Fey M F and Jeffreys A J
(1988) Genomics, 4, 328-334.

31. Jeffreys A J, Royle N J, Wilson V and Wong Z
(1988) Nature 332, 278-281.

32. Royle N J, Clarkson R, Wong Z and Jeffreys A J
(1988) Genomics, 3, 352-334

33. Royle N J, Clarkson R, Wong Z and Jeffreys A J
(1987) Human Gene mapping 9: Ninth international workshop on human gene mapping, Cytogenet, Cell Genet. 46, 685.

The invention will now be illustrated but not limited by reference to the following:

## Example 1

## MATERIALS AND METHODS

### (a) Preparation of genomic DNA, oligonucleotides and hybridization probes

Human DNA samples were provided by CEPH, Paris, or were prepared from venous blood as described elsewhere [26]. Oligonucleotides synthesised on an ABI 380B DNA synthesiser using reagents supplied by Cruachem were purified by ethanol precipitation and dissolved in water. The 5.6 kb Sau3A insert from human minisatellite clone MS32 [10] was subcloned into the BamHI site of pUC13 [27]. Similarly, the minisatellite inserts from recombinant M13 RF DNAs 33.1, 33.4 and 33.6 [5] were isolated as a 1.9 kb BamHI-EcoRI fragment, a 2.7 kb Sau3A-EcoRI fragment and a 0.7 kb BamHI-EcoRI respectively, and subcloned into pUC13 digested with BamHI plus EcoRI, to produce the plasmid series p33.1, p33.4 and p33.6. Appropriate minisatellite-containing DNA fragments were isolated from restricted plasmid DNAs by electrophoresis through 1% low gelling temperature agarose (Sea Plaque); gel slices containing DNA fragments were dissolved in water at 65° to a final concentration of 2$\mu$g/ml DNA. 10ng aliquots of DNA were labelled with $^{32}$P by random oligonucleotide priming [28].

### (b) Polymerase chain reaction

Aliquots of human DNA, diluted if necessary with 5mM Tris-HCl (pH7.5) in the presence of 0.1 $\mu$M oligonucleotide primers as carrier, were amplified in 10$\mu$l 67mM Tris-HCl (pH8.8), 16mm (NH$_4$)$_2$SO$_4$, 6.7mM MgCl$_2$, 10mM 2-mercaptoethanol, 6.7 $\mu$M EDTA, 1.5mM dATP, 1.5mM dCTP, 1.5mM dTTP, 1.5mM dGTP (Pharmacia), 170$\mu$g/ml bovine serum albumin (DNase free, Pharmacia) plus 1$\mu$M of each oligonucleotide primer and 1.5 units Taq polymerase (Anglian Biolabs). Reaction mixes in 1.5 ml Eppendorf tubes were overlaid with 40 $\mu$l paraffin oil and cycled for 1 minute at 950° 1 minute at 60° and 15 minutes at 70° on an Intelligent Heating Block (Cambio, Cambridge). Final amplification reactions were generaly chased by a final step of 1 minute at 60°, to anneal any remaining single-stranded DNA with primer, followed by an extension phase at 70° for 15 minutes.

(c) Southern blot analysis of PCR products

Paraffin oil was removed from PCR reactions by extraction with diethyl ether. Agarose gel electrophoresis of PCR products, Southern blotting onto Hybond N (Amersham) and hybridization with [32P]-labelled minisatellite probes were carried out as described previously [10], except that competitor human DNA was omitted from all hybridizations. Restriction digests and S1 nuclease digestion of PCR products were performed by diluting 5μl PCR reaction with 25μl restriction endonuclease or S1 nuclease buffer [29] and digesting for 30 minutes at 37° with 3 units restriction endonuclease of S1 nuclease (BRL) prior to gel electrophoresis.

(d) Isolation and PCR analysis of single human cells

Lymphocytes were isolated by diluting venous blood with a equal volume of 1xSSC (saline sodium citrate, 0.15M NaCl, 15mM trisodium citrate, pH 7.0), layering over Histopaque-1119 (Sigma) and centrifuging at 2000g for 10 min. Cells at the interface were diluted with 3 vol 1xSSC and banded again over Histopaque. Cells were pelleted by centrifuging at 2000g for 10 minutes, washed three times with 1xSSC, with centrifugation, and resuspended in 1xSSC to $10^4$ cells/ml.

Buccal cells were isolated by diluting 0.5ml saliva with 5ml 1xSSC and centrifuging at 2000g for 10 minutes. The cell pellet was rinsed three times with 1xSSC and resuspended to $10^4$ cells/ml.

Approximately 0.1μl aliquots of the cell suspensions were pipetted onto a siliconised microscope slide and rapidly examined at 100x magnification on an inverted microscope. Droplets containing a single nucleated cell were immediately diluted with 0.4μl 1xSSC and transferred to an Eppendorf tube using a disposable tip pipette. The microscope slide was re-examined to check that the cell had been removed with the droplet.

Cells were lysed prior to PCR either by heating or by treatment with sodium dodecyl sulphate (SDS) and proteinase K [23]. In the former case, the cell droplet was diluted with 4.5μl 5mM Tris-CHl (pH7.5) containing 0.1μM oligonucleotide primers, overlaid with paraffin oil and heated at 95° for 3 minutes prior to the addition of 5μl 2x concentrated PCR buffer/primers/Taq polymerase and amplification. In the latter case, the cell droplet was mixed with 0.5μl 5mM Tris-HCl (pH7.5), 0.1μM primers plus 1_1 5mM Tris-HCl (pH7.5), 40mM dithiothreithol, 3.4 μM SDS, 50 μg/ml proteinase K [23], overlaid with paraffin oil and digested at 37° for 45 minutes. 3μl water were added to the digest, and heated at 95° for 3 minutes to inactivate proteinase K prior to addition of 5 μl 2x PCR reaction mix as above.

(a) Selection of human minisatellites for amplification by PCR

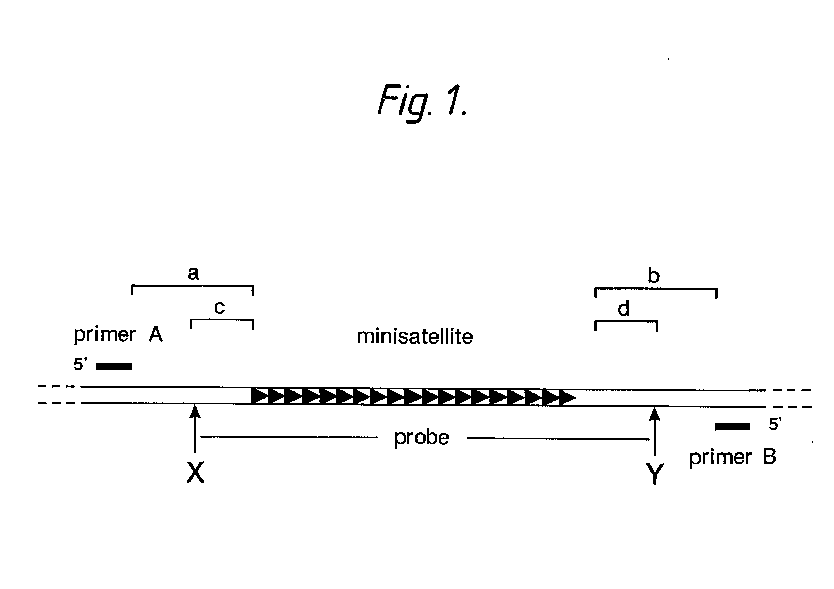

The strategy for amplifying minisatellites is shown in Figure 1. Oligonucleotide primers corresponding to unique sequence DNA flanking the minisatellite are used to drive amplification of the entire minisatellite by Taq polymerase. Amplified alleles are detected by Southern blot hybridization with a minisatellite probe located internal to the priming sites. Six cloned minisatellites were chosen for study (Table 1). Two of them, pλg3 and MS32 [8, 10], detect highly variable loci with heterozygosities of 97% and more than 40 alleles varying in the number of repeat units. The other four minisatellites, 33.1, 33.4 and 33.6 [5] and pMS51, isolated as a Sau3A-EcoRI DNA fragment cloned from a DNA fingerprint (A J Jeffreys, unpublished data), detect much less variable loci with heterozygosities of 66-77%; the alleles are however shorter than those of pλg3 and MS32 (Table 1) and should be more amenable to amplification by PCR. The flanking sequences of pλg3, 33.1, 33.4 and 33.6 have been described previously [5, 8]; the flanking DNA of λMS32 and pMS51 was sequenced as described before [8]. All flanking DNA sequences were screened against the EMBL DNA sequence database to identify repeat elements such as Alu, and PCR oligonucleotide primers A and B (Figure 1) were designed to avoid such elements. Details of all primers and hybridization probes are given in Figure 1 legend.

(b) Fidelity and efficiency of PCR amplification of human minisatellite alleles

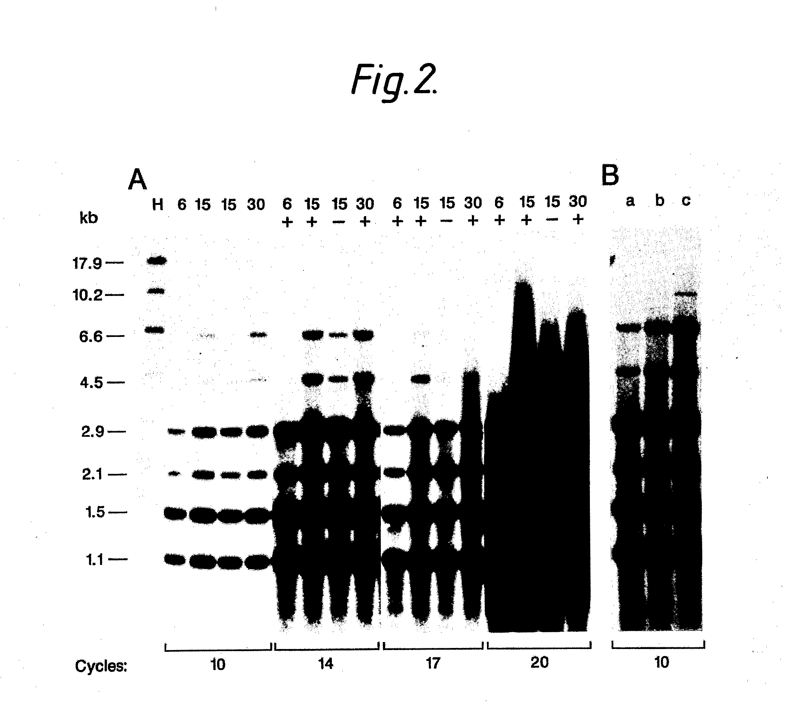

To determine the ability of Taq polymerase to amplify in particular long minisatellite alleles, a mixture of 0.1μg genomic DNA from each of 4 individuals, giving a total of 8 different MS32 alleles ranging in length from 1.1 to 17.9 kb, was amplified for 10-20 cycles using λMS32 flanking primers A and B, followed by Southern blot hybridization with a minisatellite probe (Figure 2A). Using 6 minutes extension times for Taq polymerase, only the four shortest alleles (1.1-2.9 kb) were efficiently amplified. Increasing the extension time to 15 minutes, to improve the chance that the Taq polymerase would progress completely across the minisatellite, gave a marked increase in yield of the next two larger alleles (4.5, 6.6 kb), though no further improvement was seen with 30 minutes extensions. The relative yield of large alleles could also be improved by increasing the concentration of Taq polymerase (Figure 2B), allowing the detection of a 10.2 kb allele, albeit faintly. Addition of extra Taq polymerase at the 13th cycle gave only a marginal improvement in yield, and there is no evidence for a significant drop in polymerase activity during these prolonged extension times. Further experiments varying annealing temperature, extension temperature and buffer concentration failed to improve the yield of large alleles (data not shown), and all further experiments used 15 minutes extension times and high concentration of Taq polymerase (1.5 units per $10\mu l$ PCR reaction).

At low cycle numbers (10 cycles), the alleles amplified appear to be completely faithful copies of the starting $\lambda$MS32 alleles, as judged by their electrophoretic mobilities (Figure 2A). At higher cycle numbers (14, 17 cycles), there is an increase in background labellings; since most of this can be eliminated by digestion with S1 nuclease (data not shown), much of this background probably arises from low levels of single-stranded templates from the previous cycle which have failed to prime, and from incomplete extension products from the previous cycles which by definition cannot prime. At high cycle numbers (20), the hybridization pattern degenerates to a heterodisperse smear, as expected since the yield of PCR product becomes so high (>400ng/ml) that out-of-register annealing of single-stranded tandem-repeated minisatellite DNA will occur during the extension phase. This will lead to premature termination of extension at a reannealed site, to spurious "alleles" arising from the extension of incomplete templates annealed out-of-register to the complementary strand of a minisatellite, and to the formation of multimolecular networks of reannealed ministaellite DNA strands.

The yields of each $\lambda$MS32 allele amplified by PCR were quantified by scanning densitometry (Figure 3). PCR products from 0.1 $\mu$g genomic DNA accumulate exponentially at least up to cycle 17.

The gain in product per cycle decreases monotonously with allele length, with lower gains for 6 minutes compared with 15 minutes extension times. The gain versus allele length curves extrapolate back to a gain per cycle of approximately 2.0 for very short alleles, indicating that the efficiency of denaturation and priming at each cycle is close to 100%. Final yields of an allele can be calculated from these curves; for an allele A with gain $g_A$ per cycle present initially at n molecules, the yield after c cycles is approximately $n.g_A^c$ molecules. The molar imbalance between alleles A and B of different lengths, arising through more efficient amplification of shorter alleles, is given by $(g_A/g_B)^c$. For example, after 10 cycles of amplification with 15 minutes extension times, the molar yield of a 1 kb allele will be 18 times higher than that of a 6 kb allele; after 25 cycles, the imbalance will be 1300-fold. This imbalance is ameliorated to some extent by the more efficient detection of longer alleles by the minisatellite hybridization probe. Nevertheless, long alleles amplified by PCR will become increasingly difficult to detect with low amounts of starting human genomic DNA and high numbers of PCR cycles.

Minisatellites p$\lambda$g3, pMS51, 33.1, 33.4 and 33.6 were also tested for their ability to be amplified by PCR (data not shown). In all cases, faithful amplification of all alleles tested was observed, except for the longest (> 8kb) alleles of p$\lambda$g3 which as expected failed to amplify. Again, yields of PCR product fell with increasing allele length.

## (c) Fidelity of amplification of single minisatellite molecules

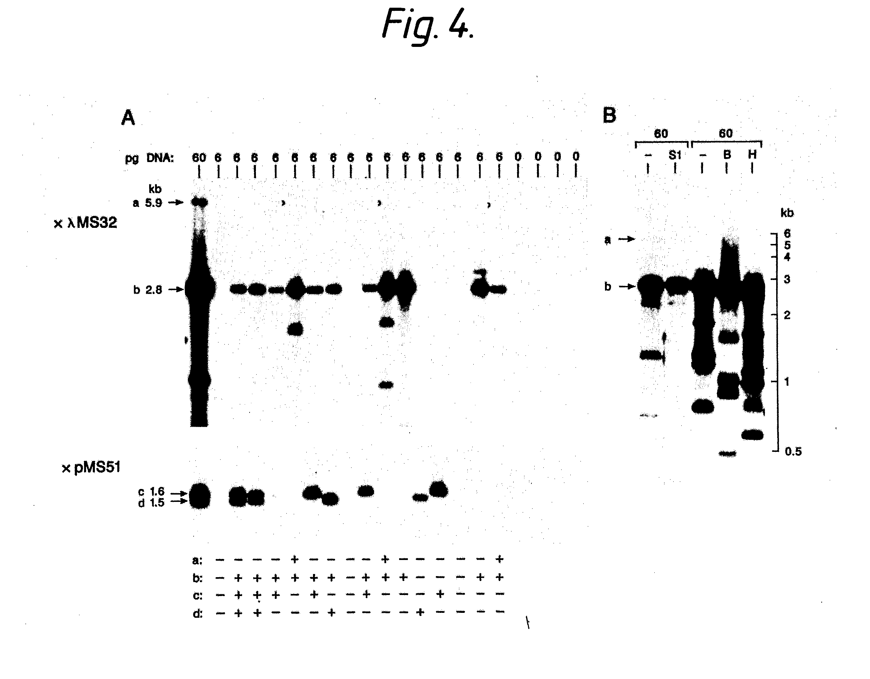

To test whether faithful amplification of single molecules is possible, 60 and 6 pg aliquots of human DNA, equivalent to 10 and 1 cells, were co-amplified for 25 cycles using primers for both $\lambda$MS32 and pMS51 (Figure 4A). Both alleles of pMS51 (1.6 and 1.5 kb) amplified in the 60pg sample, and amplification products of one or both alleles were also seen in the 6pg samples, indicating that single target molecules can be faithfully amplified. Similarly, the 2.8 and 5.9 kb alleles of $\lambda$MS32 could be successfully amplified from 6pg samples of human DNA, although the yield of the larger allele was as expected very low. Successful amplification of MS32 and pMS51 alleles in 6pg samples appeared to occur independently, as expected, with a mean failure rate per allele per reaction of 63%. From the Poisson destribution, this indicates on average 0.46 successful amplification events per 6pg DNA samples, compared with 1 event predicted since 6pg human DNA will on average contain one molecule of an allele. Thus, single target minisatellite molecules can, with reasonable efficiency, be amplified by PCR. No spurious amplification products were seen with pMS51. In contrast, MS32 frequently gave unexpected products in both the 60pg and 6pg DNA samples (Figures 4A,B). S1 nuclease digestion eliminated one band (Figure 4B) which appears only occasionally, which comigrates with denatured PCR product and· which can be largely eliminated by chasing the PCR products by a final annealing/extension step (data not shown, see Material and Methods). This S1 nuclease-sensitive band presumably corresponds to single-stranded template which failed to prime in the final PCR cycle. The remaining spurious bands detected by λMS32 were resistant to S1 nuclease but were reduced in size, along with the correct PCR product, by digestion with restriction endonucleases which cleave DNA flanking the minisatellite (Figure 4B). These spurious bands presumably represent abnormal PCR products with normal flanking DNA but altered numbers of minisatellite repeat units. They are particularly prominent after 30 cycles of amplification, are generally present in low amounts compared in the authentic allele, and vary in length from reaction to reaction, in contrast to the parent allele. They are not the result of contamination of the PCR reactions with human DNA or with products of previous PCR reactions, since they only appear in reactions where succesful amplification of an authentic allele has occurred (Figure 4A) and have been consistently seen with all human DNAs tested (data not shown). Since almost all of the spurious products are shorter than the authentic allele, it is likely that they arise fairly early in the PCR reaction and accumulate proferentially due to their short length and concommitant higher efficiency of amplification. It is not yet clear how these "mutant" alleles arise, nor whether PCR conditions can be found which will suppress their appearance. A similar frequency of appearance of abnormal "alleles" has been seen with pλg3, but only much less frequently with the other four minisatellites tested.

Somatic mutations at ministatellite loci in the starting human genomic DNA could also be a source of unexpected PCR products. Such mutations do exist, particularly for λMS32, as shown by the appearance of mutant minisatelllite alleles in clonal tumour cell populations [30]. Somatic mutants are however unlikely to be a major source of the spurious bands shown in Figure 4, since no PCR reactions on 6pg human DNA have yet been seen which show a mutant allele appearing in the absence of the normal parental allele.

## (d) Co-amplification of multiple minisatellites: PCR-derived human DNA fingerprints

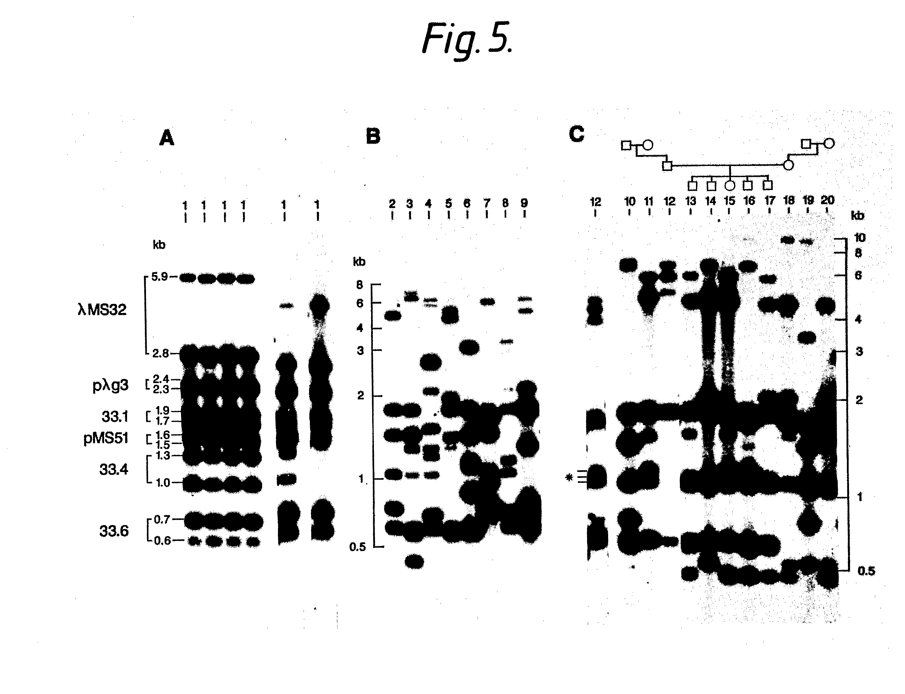

Figure 4 demonstrates that two minisatellites can be successfully co-amplified in the same PCR reaction. Further analyses showed that at least six minisatellites could be co-amplified without any apparent interference between loci. Furthermore, the PCR products could also be typed simultaneously by Southern blot hybridization with a cocktail of all six minsatellite probes. Examples of such multilocus PCR-derived DNA "fingerprints" are shown in Figure 5. In all cases, the PCR reaction was limited to 15-18 cycles, to minimise the appearance of spurious products as seen in Figure 4. Repeat analyses of the same individual showed that the pattern was reproducible, with all hybridizing DNA fragments representing authentic minisatellite amplification products. DNA "fingerprints" could be readily derived from 1ng human DNA; On occasion, one or two loci failed to amplify (individuals 1, 12, Figure 5A, C); this failure usually affected 33.4, followed by pMS51, and was least likely to affect 33.1 (data not shown). The likelihood of failure appears to correlate with the GC content of the minisatellite repeat units (Table 1), and suggests that non-amplification results from failure to denature GC-rich minisatellites at 95°, probably due to localised variations of temperature in the heating block or to poor thermal conductivity between the block and the reaction tube.

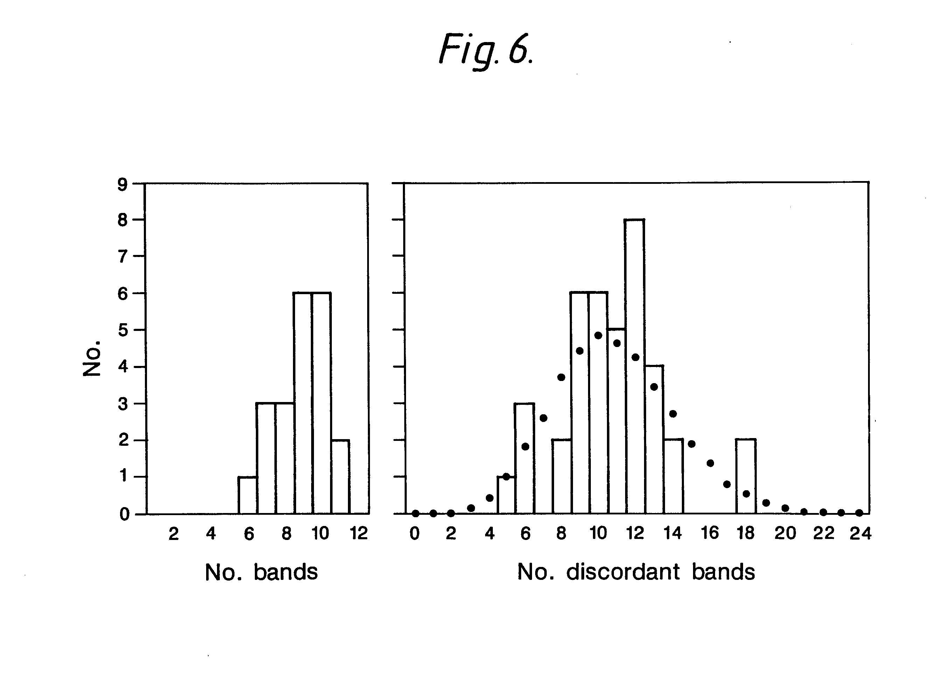

These PCR DNA fingerprints are derived from six loci with widely differing levels of variability (Table 1). To determine the overall complexity and level of variability of these patterns, unrelated individuals were compared (Figure 5B). On average, 8.9 bands were resolved per individual (range 6-11, Figure 6). The maximum possible number of bands is 12 (Figure 5A), corresponding to heterozygosity at all loci, with no electrophoretic comigration of alleles from different loci and with·no alleles too large to be amplified by PCR. In pairwise comparisons of unrelated individuals, there are on average 10.8 bands which are discordant between pairs of individuals (range 5-18). Since the distribution of discordancies approximates to a Poisson distribution, then the chance that two unrelated individuals would show identical DNA fingerprints (no discordant bands) can be estimated at $e^{-10.8} = 2 \times 10^{-5}$. These patterns therefore show a good degree of individual-specificity, despite the fact that four of the six loci used show relatively modest levels of variability (Table 1). Differences in PCR-derived DNA fingerprints are also readily detectable between closely related individuals, in for example the 3-generation family shown in Figure 5C in which faithful transmission of bands from parent to offspring can also be seen.

## (e) Typing minisatellites in single human cells

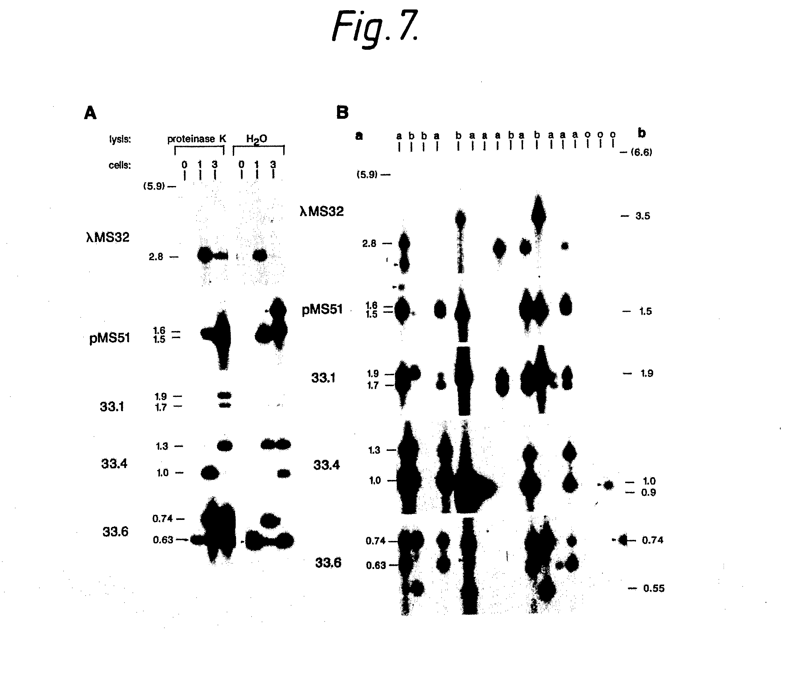

Lysed cells can be subjected directly to PCR without the need for purifying DNA [23], which greatly simplifies the analysis of specimens such as blood. In preliminary experimaents it proved possible to type minisatellites in blood by first freezing and thawing blood to lyse erythrocytes, followed by centrifugation to collect white cells and nuclei, and heating in water to lyse cells prior to PCR. Using this method, it was possible to reproducibly type 0.001-0.01 $\mu$l blood, corresponding to 3-30 nucleated cells (data not shown). This analysis was extended to single lymphocytes (Figure 7A) from which five minisatellite loci were simultaneously co-amplified and typed by sequential hybridization. Successful and reasonably faithful amplification was seen both from cells lysed with proteinase K and SDS prior to PCR, and from cells lysed by heating in water. Individual nucleated buccal cells could also be typed following proteinase K/SDS lysis (Figure 7B), though these cells failed to lyse in water (data not shown).

To test the feasibility of identifying individual cells, 14 buccal cells from two individuals were separately typed in a blinded experiment (Figure 7B). In four cases, no amplification products were seen from any of the loci, suggesting either that the cell had not been transferred to the PCR reaction, or that lysis had not occurred, or that nuclear DNA had degraded prior to PCR. In the remaining 10 cases, amplified alleles could be detected from at least two of the minisatellite loci, and in some cases all five loci amplified successfully from a single cell. Omitting the large alleles of MS32, which amplify poorly and would be difficult to type at the single cell level, we estimate that, for those single cell PCR reactions in which at least some loci have amplified, approximately 75% of alleles present could be detected following PCR. This estimate agrees with the efficiency of single molecule amplification determined from PCR analysis of 6pg sample of human DNA (Figure 4). As expected from Figure 4, several instances of spurious bands were seen in both bucccal cell and lymphocyte PCR reactions (Figure 7A,B). Nevertheless, distinguishing alleles from each of the two individuals tested could be detected in the 10 successfully-typed buccal cells, and the origin of each buccal cell was successfully predicted in this blinded trial.

Finally, we note the presence of amplified products of 33.6 in some of the DNA-free controls in Figure 7A, B and 33.4 in one of the buccal cell controls. In practice, we have found that such contamination, probably with recombinant DNAs or the products of previous PCR reactions rather than with human cells or genomic DNA, can only be avoided by using solutions, glassware, disposable pipette tips and Eppendorf tubes which have not previously been exposed to the laboratory environment. It is noteworthy that our most consistent contamination problem has been with 33.6, one of the multilocus DNA fingerprint probes [5,6] which have been in continuous use in our laboratory for the last four years.

## (f) CONCLUSIONS

Taq polymerase not only shows remarkable fidelity in amplifying non-repeated DNA [21], but is also capable of faithfully amplifying entire minisatellites and preserving the allelic specificity of the number of repeat units. Unlike conventional PCR reactions, however, minisatellite PCR usually has to be terminated before the yield of product becomes so high (>4ng per 10$\mu$l PCR reaction) that out-of-register annealing between complementary tandem-repeated template strands occurs during primer extension, particularly during the lengthy extension times needed to obtain efficient amplification of long minisatellite alleles. Also, the PCR reaction must proceed far enough to generate sufficient product to be detectable by hybridization. The minisatellite probes are sensitive and can readily detect 0.1pg minisatellite PCR product [10]. The "window" of PCR cycles which generate an appropriate amount of product for typing (0.1-4000 pg product) is therefore very wide, and only a very approximate estimate of the amount of initial human genomic DNA is needed to predict the number of PCR cycles needed for successful typing. As a guide, 10-15 cycles are appropriate for 100ng genomic DNA, 18 cycles for 1ng and 25 cycles for single cell PCR (6pg). The number of PCR cycles may need to be increased to detect larger alleles which amplify less efficiently.

Since minisatellite amplifications often need to be restricted to the exponential phase of accumulation of PCR products [21], then the hybridization signal is approximately proportional to the amount of input DNA down to 0.1ng human genomic DNA (data not shown). Below this level, stochastic variation in the number of target minisatellite molecules can obscure the proportionality. Minisatellite PCR can therefore be used quantitatively to estimate low concentrations of human DNA. Also, the amount of primers and Taq polymerase will not be limiting during this early phase of PCR, and in principle there should be little or no interference between different loci being amplified. In practice, at least six different minisatellites can be co-amplified simultaneously, and there seems to be no theoretical reason why this number could not be increased further.

Co-amplification of minisatellites followed by simultaneous or sequential hybridization with minisatellite probes enables a considerable amount of information concerning individual identity and family relationship to be gathered from very small DNA samples. These PCR-derived DNA fingerprints appear to be reliable

down to 1ng human DNA. Information can also be recovered from much lower amounts of DNA and single cells, although the generation of spurious DNA fragments at some loci, during the relatively large number of PCR cycles needed for single cell typing, could present significant problems for individual identification at the level of one or a few cells. Fortunately, these spurious PCR products appear to vary from reaction to reaction, and duplicate PCR analyses of very small samples of DNA should therefore distinguish bona fide amplified alleles from spurious PCR products.

PCR-derived DNA fingerprints already show a good level of individual specificity, with a chance of false association of two individuals of approximately $2 \times 10^{-5}$. In contrast, conventional DNA fingerprints obtained by Southern blot hybridization with a multilocus polycore probe [6] or with a cocktail of locus-specific human minisatellite probes [10] show much higher levels of individual specificity ($<10^{-12}$ and $<10^{-6}$ respectively). However, the variability of PCR-derived DNA fingerprints could be improved substantially. First, highly informative alleles particularly at pλg3 and λMS32 cannot be detected above approximately 8 kb (1ng human DNA) or approximately 5 kb (single cell). This problem could be overcome by using highly variable minisatellites with a more restricted range of allele lengths. Such loci appear to be scarce since high levels of variability are usually associated with large numbers of minisatellite repeat units and long alleles [5, 8, 10]. Some possibly appropriate loci have however been isolated [9], J.A.L.Armour and A.J. Jeffreys, unpublished data). Second, the number of minisatellites being amplified simultaneously could be increased. Third, loci which are particularly prone to generate spurious PCR products, such as pλg3 and MS32, could be identified and avoided. If these goals can be accomplished, then we see no reason why reliable identification at the single cell level should not be possible, provided that inadvertant contamination is avoided and that the potential presence of somatic mutations at hypervariable loci is taken into account [30].

The use of multilocus DNA fingerprint probes in for example individual identification in forensic medicine, paternity testing and monitoring bone marrow transplants is limited by the sensitivity of these probes which require at least 0.1-1μg human DNA for typing [6]. Similarly, locus-specific minisatellite probes can only type down to approximately 50ng human DNA [10]. PCR-derived DNA fingerprinting improves sensitivity by orders of magnitude and can be used to type specimens which are relatively intractable by conventional Southern blot hybridization. For example, human hair roots typically contain 10-500ng DNA [22] and while approximately 70% of roots can be typed using locus-specific minisatellite probes (Z, Wong, J.A.L. Armour and A.J. Jeffreys, unpublished data), all hair roots so far tested can be typed by PCR-derived DNA fingerprinting (data not shown). Similarly, 0.001-0.01μl blood can be typed without the need first to purify DNA. Likewise, saliva contains typically 100-1000 nucleated buccal cells per μl, and PCR-derived DNA fingerprint analysis of submicrolitre samples of saliva is therefore possible. The potential for typing trace amounts of hair, blood, semen, saliva and urine in forensic specimens, including partially degraded samples, is obvious. The potential for inadvertant contamination of specimens, for example with traces of spittle, is likewise evident.

PCR-derived DNA fingerprints should eventually become sufficiently individual-specific to provide a highly polarised test for establishing parentage in for example paternity disputes. Not only would the need to isolate DNA be obviated, but much smaller samples of blood obtained by finger-pricking rather than venipuncture could be used. Alternatively, the determination of parentage could be based on the analysis of saliva. This would avoid the problem of individuals who object to giving blood samples on religious or other grounds, and would remove the trauma of taking blood from infants.

## Example 2

### MATERIALS AND METHODS

#### Amplification of minisatellite alleles by PCR

Oligonucleotide primers were prepared as described previously (Jeffreys et al, Nucleic Acids Res. 16, 10953-10971). λMS32 minisatellite alleles were amplified by mixing 0.4μg human genomic DNA in <4 1 H₂O with 50μl 45mM Tris-HCl (pH8.8), 11mm $(NH_4)_2$ $SO_4$, 4.5mM $MgCl_2$, 4.5μM EDTA, 7mM 2-mercaptoethanol, 1mm dATP, 1mm dCTP, 1mM dGTP, 1mM dTTP (Pharmacia), 110μg/ml bovine serum albumin (DNase free, Pharmacia), 1μM oligonucleotide primer A and 1μM oligonucleotide primer B. 7.5 units Taq polymerase (Cambio type III or Perkin Elmer AmpliTaq) were added, the reaction overlaid with

40μl paraffin oil, and cycled at 95° for 1.5 minutes, 67° for 1.5 minutes and 70° for 9.9 minutes for 30 cycles on a Techne Programmable Dri-Block PHC-1, followed by a final chase at 67° for 1.5 minutes and 70° for 9.9 minutes. Equivalent results were obtained on a Perkin Elmer Cetus DNA Thermal Cycler, cycling at 95° for 1 minute, 67° for 1 minute and 70° for 10 minutes.

Isolation of amplified alleles

50_1 PCR reactions were mixed with 5_1 loading mix (12.5% ficoll 400, 0.2% bromophenol blue, 0.2M Tris acetate (pH8.3), 0.1M Na acetate, 1mM EDTA), loaded onto a 1% agarose gel (Sigma Type I) and electrophoresed in the presence of ethidium bromide. Amplified alleles were visualised using a long wavelength u/v wand (or a short wavelength u/v transilluminator if necessary) and gel slices containing alleles were excised. DNA was recovered by electrophoresis onto dialysis membrane, ethanol precipitated and dissolved in 10μl $H_2O$.

Amplification

We have previously described how minisatellite alleles can be amplified using oligonucleotide primers corresponding to DNA flanking the minisatellite to drive the amplification of the entire tandem repeat array. By limiting the number of PCR cycles and detecting amplified alleles by Southern blot hybridization with a minisatellite probe, we showed that faithful amplification was possible, but that at high cycle numbers the products collapsed to a heterodisperse smear of amplified products due to annealing between single-stranded tandem-repeated minisatellite DNA fragments. Also, most of the PCR products detectable at high cycle numbers on an ethidium bromide stained agarose gel did not correspond to PCR products of the minisatellite locus but arose by mispriming at other genomic sites (A.J. Jeffreys and V. Wilson, unpublished data).

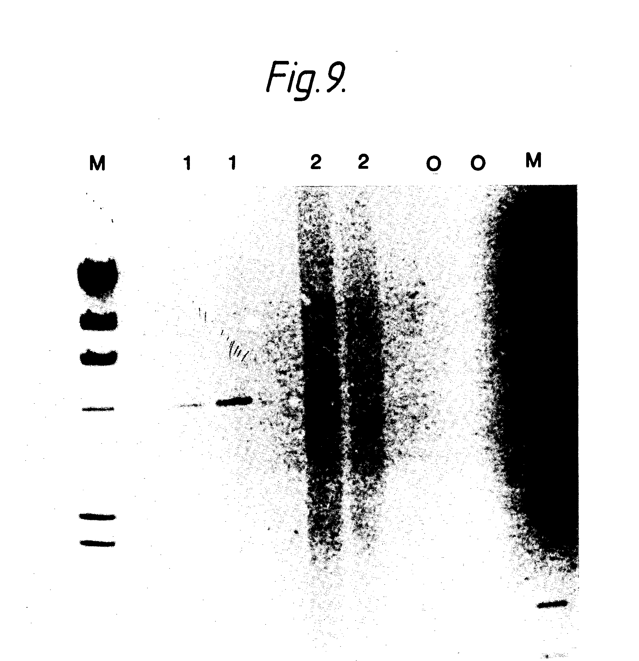

By decreasing the ionic strength of the PCR buffer and increasing the PCR annealing temperature to reduce mispriming, as well as changing the source of the Taq polymerase, it has proved possible to amplify minisatellites to the point where authentic PCR-amplified alleles can be directly visualised on an ethidium stained gel (Figure 9). Some background smearing which resolves into discrete low molecular weight DNA fragments does occur, as a result of the large number of PCR cycles needed to generate this amount of product which produces, by annealing, heterodisperse minisatellite products. Nevertheless, authentic alleles are clearly identifiable and correspond in size to the minisatellite alleles detected by conventional Southern blot hybridization of human genomic DNA.

As noted previously (Jeffreys et al., 1988), yields of minisatellite alleles fall with allele length, and it has not yet proved possible to amplify minisatellites longer than 6 kb to the point where they can be identified on an ethidium-stained gel. For 50μl PCR reactions containing 0.4μg human genomic DNA and processed for 30 cycles, up to 1μg small (1 kb) alleles can be produced; this yield falls to = 1ng for 6 kb alleles. In heterzygotes with widely differing allele sizes, over-amplification of the smaller allele usually causes the premature collapse of the larger allele and its disappearance from ethidium stained gels.

To map minisatellite variant repeats (MVR) in minisatellites, the amplified allele is recovered by preparative gel electrophoresis, cleaved with a restriction endonuclease which cuts close to one or other end of the amplified allele, and end-labelled by fill-in labelling at the cleaved end. Partial digestion of the end-labelled fragment with appropriate restriction enzymes followed by agarose gel electrophoresis and authoradiography generates a map of the location of internal MVRs. Terminal cleavage can be carried out using an appropriate restriction site lying in flanking DNA within the region being amplified, or alternatively one of the primers can span a suitable restriction site. The 5′primer for λ MS32, 5′-TCACCGGTGAATTCCACAGACACT-3′, contains an EcoRI site 9-14nt within the primer, corresponding to an EcoRI site in genomic DNA. In the absence of an appropriate site in DNA flanking the minisatellite, as is the case with the region 3′ to λ MS32, a terminal site can be generated by using a 5′ extended oligonucleotide primer. Thus the second primer used to amplify λMS32 has been modified to 5′-CTGATCATCGATCGACTCGCAGATGGAGCAATGGCC-3′, where the underlined region corresponds to genomic DNA sequences; the 5′ extension contains a site for ClaI (ATCGAT) which does not cleave near λMS32. The presence of the 5′ extension has no effect on the ability of this primer to amplify λMS32 (data not shown). Alleles amplified with these two primers can be labelled at either end following cleavage with EcoRI or ClaI, allowing MVR mapping to be carried out from either end of the tandem-repeat array.

Mapping MVRs within amplified MS32 alleles

Screening the CEPH panel of families of Mormon, French, Amish and Venezuelan origin has revealed AluI alleles of λMS32 ranging from 1.2kb to 20kb long; since AluI alleles are only = 0.2 kb longer than the corresponding PCR products with the above primers, then AluI alleles longer than 6.2 kb cannot easily be mapped internally due to low amplification efficiency.

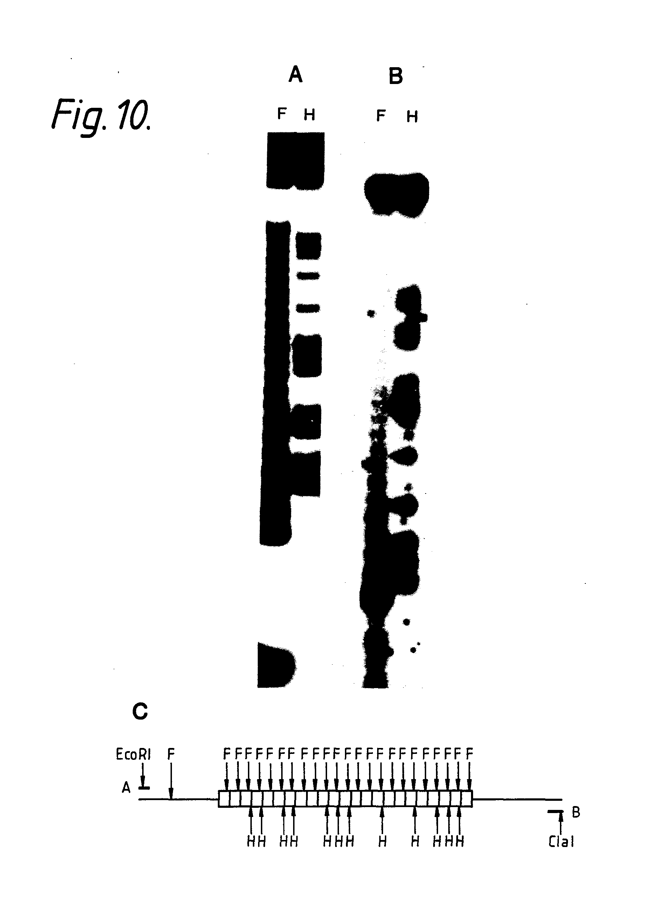

Internal mapping results for the shortest allele in the CEPH panel are shown in Figure 10 for the EcoRI and ClaI labelled amplified allele. Both produce a continous HinfI ladder and a discontinuous HaeIII ladder, the latter being complementary for EcoRI and ClaI. A complete map of the location of all MVRs cleavable by HaeIII can be deduced. Repeat analysis of this allele amplified on separate occasions produced the same map (data not shown), indicating fidelity of amplification and mapping.

Example 3

Single Molecule MVR Mapping

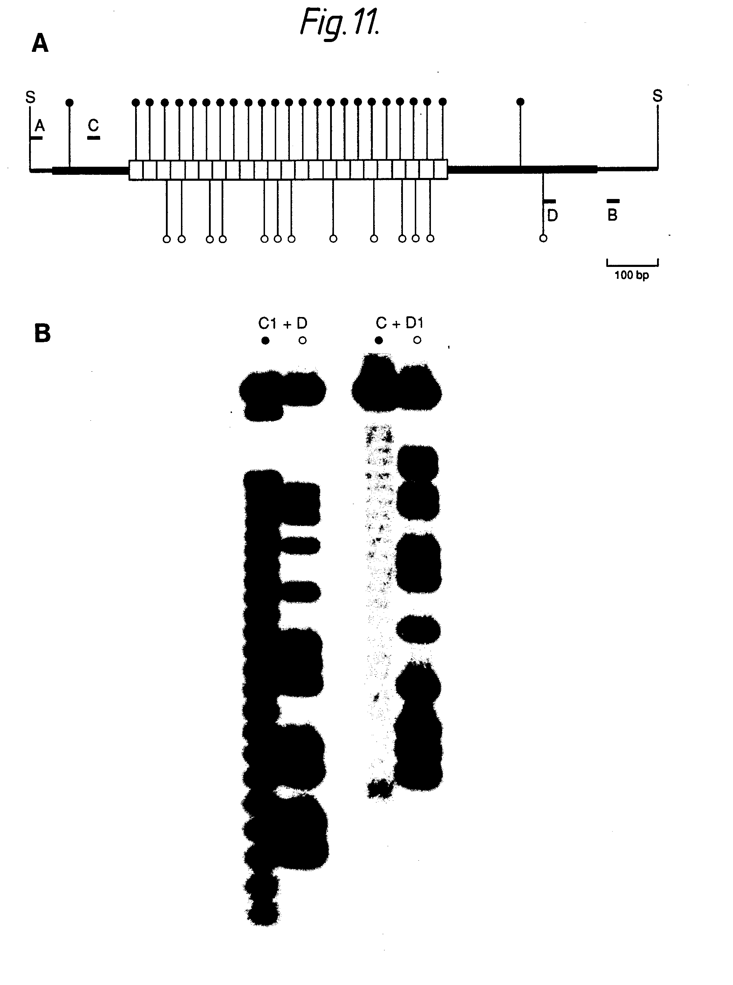

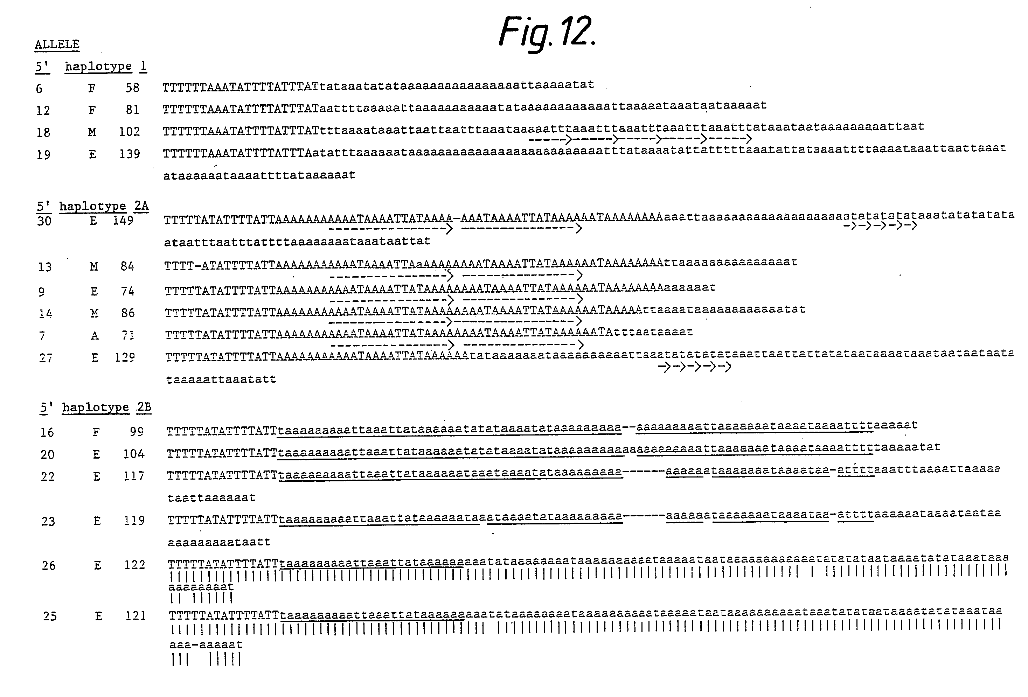

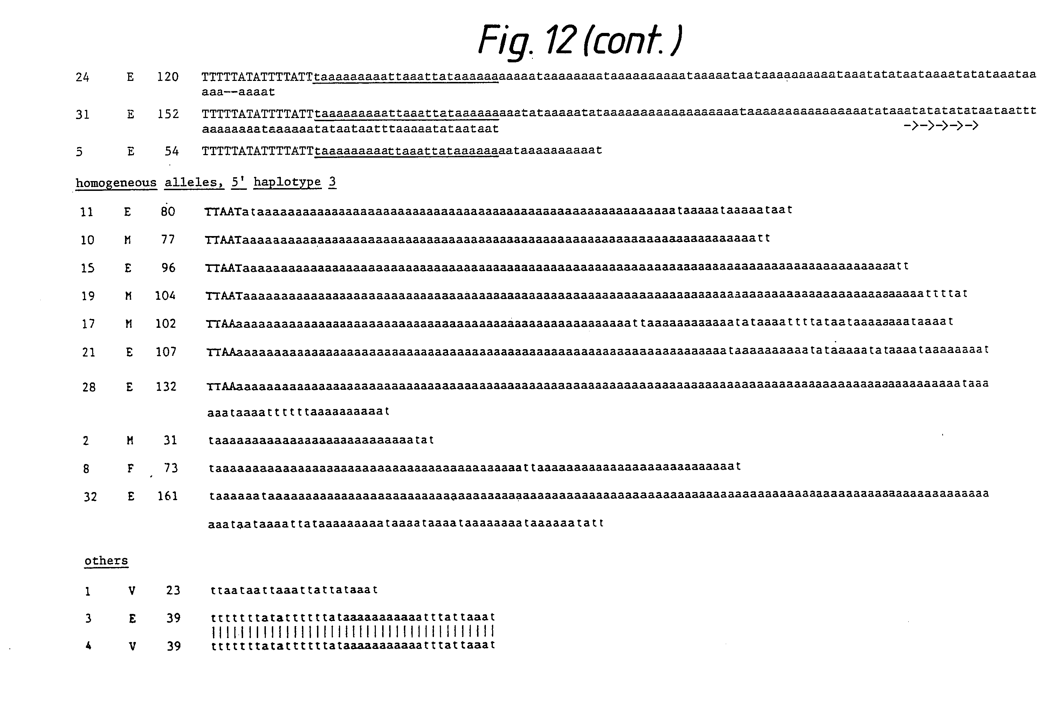

We have shown that single minisatellite molecules can be amplified with reasonable efficiency and fidelity, although with the occasional appearance of spurious PCR products of incorrect length (Jeffreys et al., 1988). Use of the modified PCR conditions described here has almost completely suppressed the appearance of spurious PCR products even from long alleles (Figure 12A). To determine the fidelity of single molecule MVR mapping, 16 aliquots of 6 pg DNA from CEPH individual 10208 heterozygous for alleles q and 4 (Figure 12A) were amplified for 25 cycles with external primers A and B (Figure 11A) which are reserved. exclusively for single molecule analysis to minimize the risk of carry-over contamination. Southern blot hybridization with an MS32 minisatellite probe revealed amplified alleles corresponding in length to allele 1 in 5 samples, and allele 4 in 6 samples. No other amplified products were detected, and parallel zero DNA controls were also negative (data not shown). From these data, 42% of single minisatellite molecules give rise to PCR products, an efficiency similar to that reported previously (Jeffreys et al., 1988) and similar to the efficiency of amplification of larger alleles (Figure 12A legend). The 11 alleles amplified from single molecules were re-amplified with the nested primers C1 and D, to minimize subsequent contamination of single molecule PCR reactons and to eliminate spurious products arising from mispriming with primers A and/or B elsewhere in the genome, and subjected to internal mapping. All maps obtained were indistinguishable from the maps of alleles 1 and 4 obtained by amplification from bulk genomic DNA (data not shown).

Amplification of new delection mutants of MS32 from size-selected human genomic DNA

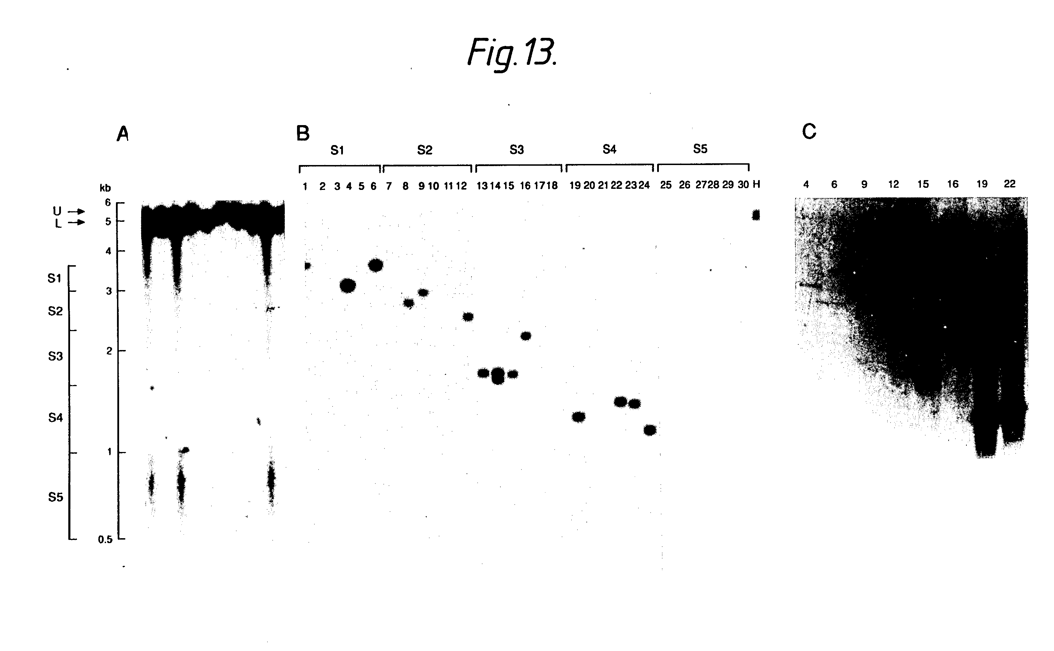

The ability to amplify faithfully single molecules of MS32 make it possible to amplify and characterize de novo mutations at this locus present in bulk human genomic DNA. An individual was chosen who was heterozygous for alleles of similar length but very different internal structure (alleles 31 and 32, Figure 12). PCR analysis on 40 duplicate aliquots of 30 pg sperm DNA from this individual showed efficient amplification of single minisatellite molecules with no evidence for additional abnormal length alleles in any reaction (Figure 13A).

Sperm DNA from this individual was digested with Sau3A plus the isoschizomer MboI which cleave distal to primers A and B (Figure 11A), and size fractionated by agarose gel electrophoresis. A series of fractions containing DNA fragments smaller than the progenitor alleles 31 and 32 were collected, and aliquots of each fraction amplified with primers A plus B. Southern blot hybridization revealed single molecule amplification events in these fractions, giving discrete PCR products whose size fell within the size range of the DNA fractions tested (Figure 13B, Table 2). Multiple parallel control PCR reaction containing no DNA, or containing aliquots of equivalent size fractions from a control Sau3A/MboI digest lacking sperm DNA (see Experimental Procedures), were consistently negative for MS32 PCR products. The smallest size fractions S3-S5 from sperm DNA were completely devoid of amplifiable precursor alleles, indicating that >99.9999% of precursor allele molecules has been eliminated from these fractions. Trace amounts of these alleles were still present in fraction S1 and S2 indicating incomplete purification despite repeated rounds of gel electrophoretic fractionation. Gel purification of DNA close in size to the progenitor alleles yielded more heavily contaminated fractions in which the detection of new mutant alleles was hindered by PCR products

38

from the progenitor alleles (data not shown).

Single molecule minisatellite deletion mutants were similarly amplified from size-fractionated DNA prepared from blood taken from the same indivdiual. Again, single molecule amplification events were readily detectable in blood DNA, but not in control digests lacking blood DNA. Details of the number and sizes of new mutant sperm and blood alleles detected are summarized in Table 3. A total of 64 sperm and 42 blood mutants were observed, with mutants occurring most frequently in the largest size fractions.

The ability to align all sperm and blood mutants with one or other progenitor allele provides strong supporting evidence that these short alleles represent bona fide mutant molecules present in genomic DNA, rather than contaminants introduced during the isolation of sperm and blood DNA. Furthermore, the repeated detection of identical mosaic mutant alleles further confirms the fidelity of single molecule MVR mapping.

## EXPERIMENTAL PROCEDURES

### Preparation of DNA

Human DNAs were provided by CEPH, Paris, and were also prepared from venous blood collected from 80 unrelated English individuals as described elsewhere (Jeffreys and Morton, 1987). Blood DNA for single molecule PCR analysis was prepared from 20ml blood collected into 20ml 1 x SSC (saline sodium citrate, 0.15 NaCl, 15mM trisodium citrate pH 7.0). The blood was frozen and thawed to lyse erythrocytes, and leucocytes collected by centrifugation at 10,000g for 10 minutes. The cell pellet was rinsed with 1 x SSC, resuspended in 0.2M Na acetate (pH 7.0) and lysed by the addition of SDS to 1%. DNA was collected, following phenol extraction, by three rounds of ethanol precipitation. All operations were performed in a laminar flow hood using pipettes, disposable plasticware and reagents which had not been previously exposed to the laboratory environment, to minimize the risk of contamination. Sperm DNA was similarly prepared from a single ejaculate from an individual who had avoided sexual intercourse prior to giving the sample (to prevent contamination with DNA from his partner). Semen was diluted with an equal volume of 1 x SSC and sperm collected by centrifugation at 10,000g for 10 minutes. The cell pellet was rinsed three times with 20ml 1 x SSC, with centrifugation, followed by six washes with 20ml 1 x SSC, 1% SDS to lyse any seminal leucocytes and epithelial cells. The residual sperm pellet was incubated in 1 x SSC, 1M 2-mercaptoethanol at room temperature for 5 minutes, and the reduced sperm lysed by the addition of SDS to 1%. Sperm DNA was collected after phenol extraction by ethanol precipitation.

### Size fractionation of human DNA

40$\mu$g blood sperm DNA was digested to completion with Sau3A plus Mbol in the presence of 4mM spermidine trichloride using conditions recommended by the manufacturer. The DNA digest, together with a control digest lacking DNA, was loaded into a horizontal 20cm long 0.6% agarose gel (Sigma type 1) in TNE (20mM Tris acetate, 10mM Na acetate, 0.1mM EDTA, pH 8.3) such that the loading slot was less than half filled with sample. Samples were electrophoresed alongside Hind III DNA markers at 20V for 18 hours. The marker tracks were removed and stained with ethidium bromide. The gel region containing human DNA fragments ranging from 0.6 - 4.2kb was excised, along with the corresponding region from the control digest, and DNA collected by electro-elution onto dialysis membrane. The recovered DNA, plus the control extract, was refractionated as above on a fresh gel. Recovered DNA was then electrophoresed through a third gel, and a series of gel slices from 0.6 - 3.8 kb taken. The top and bottom 1mm of gel was trimmed from each slice to minimize contamination with DNA framgents migrating aberrantly along the gel surface, and DNA collected by electroelution and ethanol precipitation. DNA was dissolved in 5mM Tris-HCl (pH 7.5). All operations were performed in a laminar flow cabinet, and electrophoresis equipment was decontaminated prior to use by immersion in domestic bleach plus 1M HCl in a fume hood for 16 hours.

### Amplification of MS32 alleles

Alleles were amplified by a modification of previously described methods (Jeffreys et al, 1988). 0.4$\mu$g human genomic DNA was amplified in 50$\mu$l 45mM Tris-HCl (pH 8.8), 11mM $(NH_4)_2SO_4$, 4.5mM $MgCl_2$,

6.7mM 2-mercaptoethanol, 4.5μM EDTA, 1mM dATP, 1mM dCTP, 1mM dGTP, 1mM dTTP (Pharmacia), 110 μg/ml bovine serum albumin (DNase free, Pharmacia) plus 1μM each of oligonucleotides C1 plus D, or C plus D1, and 5 units Taq polymerase (Amersham). Reactions were cycled for 1.3 minutes at 96°, 1 minute at 67° and 4 minutes at 70° for 25 - 30 cycles on a DNA Thermal Cycler (Perkin Elmer Cetus). PCR products were electrophoresed through a 0.8% agarose gel, and amplified alleles visualised by staining with ethidium bromide. Alleles were recovered by electro-elution and ethanol precipitation and dissolved in 5mM Tris-HCl (pH 7.0). Other sources of Taq polymerase were equally effective (AmpliTaq (Perkin Elmer Cetus) and Type III Taq polymerase (Cambio).)

Human DNA samples for single molecule analysis were centrifuged to remove any particulate matter, diluted with 5mM Tris-HCl (pH 7.0) plus 0.1μm PCR primers as carrier, and amplified in 7μl PCR reactions using primers A plus B at 96° for 1.3 min, 64° for 1 minute and 70° for four minutes for 25 - 28 cycles. PCR products were detected by Southern blot hybridization with a 32p-labelled MS32 minisatellite probe as described previously (Jeffreys et al., 1988) PCR products were re-amplified by seeding a 30μl PCR reaction containing 1μM nested primers C1 Plus D, or C plus D1, with 0.4μl of the initial PCR reaction and cycling at 96° for 1.3 minutes, 65° for 1 minute and 70° for 4 minutes for 25-30 cycles. Re-amplified alleles were purified by agarose gel electrophoresis as described above.

TABLE 1

| Properties of human minisatellites selected for PCR amplification. | | | | | | | |
|---|---|---|---|---|---|---|---|
| Clone | Locus | Chromosome localisation | Heterozygosity (%) | No alleles | Allelic length range (kb) | %GC[2] | Ref |
| p g3 | D7522 | 7q36-qter | 97 | >40 | 0.6-20 | 66 | [8, 10, 32] |
| MS32 | D1S8 | 1q42-q43 | 97 | >40 | 1.1-20 | 62 | [10, 32] |
| pMS51 | D11S97 | 11q13 | 77 | 9 | 1.3-4.3 | 69 | [33] |
| p33.1 | - | - | 66 | 10 | 1.1-2.5 | 56 | [5] |
| p.33.4 | - | - | 71 | 7 | 0.8-1.3 | 68 | [5] |
| p33.6 | - | - | 67 | 8 | 0.5-1.0 | 70 | [5] |
| 1; lengths of alleles include both the minisatellite and the flanking DNA defined by the PCR primers (see Figure 1). | | | | | | | |

2; GC content of the minisatellite repeat units. The pMS51 minisatallite repeat unit is 5′-ACATGGCAGG(AGGGCAGG)$_n$TGGAGGG-3′, where n = 1 or 2 depending on the repeat unit.

Table 2

| Properties of minisatellite loci detected by locus-specific clones | | | | | |
|---|---|---|---|---|---|
| probe | locus | per cent heterozygosity | chromosomal location | bp 5′ | flanking DNA sequenced 3′ |
| P g3 | D7S22 | 97.4 | 7q36-qter | 438 | 309 |
| pMS1 | D1S7 | 99.4 | 1p33-p35 | 1284 | 434 |
| pMS31 | D7S21 | 98.0 | 7p22-pter | 399 | 12 |
| pMS32 | D1S8 | 97.5 | 1q42-q43 | 207 | 429 |
| pMS43 | D12S11 | 95.9 (A) | 12q24.3-qter | 14 | 780 |
| | | 30 (B) | | (780) | 373 |
| pMS51 | D11S97 | 77 | 11q13 | 113 | 194 |
| pMS228 | - | 94 (A) | 17p13-pter | - | - |
| | | 85 (B) | | 400 | 1057 |
| Note: in pMS228 only DNA flanking 228B has been sequenced (see Figure 1). Restriction mapping suggests that only about 500bp immediately flanking 228A have thereby been omitted. | | | | | |

Table 3

| Summary of deletion mutants of MS32 detected in size-fractionated sperm and blood DNA digested with Sau 3A plus MboI | | | | | | | |
|---|---|---|---|---|---|---|---|
| Fraction | Size range, kb | No. haploid genomes analysed x10$^{-6}$ | No. mutants | No. mapped | No. different | No. repeats | |
| | | | | | | expected | observed |
| Sperm S1 | 3.0-3.5 | 0.16 | 17 | 12 | 6 | 83-100 | 86-118 |
| S2 | 2.4-3.0 | 1.1 | 27 | 17 | 7 | 62-83 | 59-76 |
| S3 | 1.7-2.4 | 2.9 | 15 | 4 | 3 | 37-62 | 48-60 |
| S4 | 1.1-1.7 | 3.7 | 5 | 5 | 3 | 17-37 | 25-34 |
| S5 | 0.6-1.1 | 3.4 | 0 | - | - | 0-17 | - |
| Blood B1 | 3.1-3.8 | 0.12 | 11 | 8 | 7 | 86-110 | 91-113 |
| B2 | 2.3-3.1 | 0.72 | 15 | 15 | 13 | 59-86 | 61-86 |
| B3 | 1.6-2.3 | 1.0 | 11 | 9 | 5 | 34-59 | 38-62 |
| B4 | 1.1-1.6 | 1.5 | 5 | 5 | 5 | 17-34 | 20-29 |
| B5 | 0.6-1.1 | 1.5 | 0 | - | - | 0-17 | - |

Table 3 legend

Sperm and blood DNA from an individual heterozygous for MS32 Sau3A alleles 4.9 and 5.1 kb long (alleles 31 and 32, Figure 12) were digested with Sau3A plus MboI and size-fractionated by agarose gel electrophoresis as described in Experimental Procedures. The yield of DNA in each fraction was estimated by electrophoresing aliquots of each fraction against a dilution series of unfractionated digested DNA, followed by scanning densitometry of the appropriate molecular weight region on photographs of an ethidium bromide stained gel. Multiple aliquots of each fraction were amplified using PCR primers A and B and tested for new mutant alleles as described in Figure 13. The number of haploid genomes analysed for mutants of a given size class was derived from the yield of each fraction, assuming 3pg DNA per haploid genome and corrected for the estimated proprotion (40%) of single MS32 molecules which successfully amplify. The observed number of repeats in each mutant molecule was estimated from the size of the initial product amplified with primers A plus B, and confirmed by direct determination of the number of repeats in mutants subjected to internal mapping.

Figure 1

Primers and hybridization probes used in the amplification of minisatellites by PCR. Each minisatellite locus was amplified using 20- or 24-mer primers A and B located in unique sequences flanking DNA a and b bp respectively from the minisatellite. PCR products were detected by hybridization with an internal minisatellite probe isolated by cleavage with restriction endonucleases X and Y which cleave c and d bp from the minisatellite. Details for the six minisatellites are as follows, where R = length of repeat unit (bp): p g3, A 5'-ACCACAGGCAGAGTAAGAGG-3', B = 5'-CCACCCTGCTTACAGCAATG-3', X = Pst I, Y = DdeI, a = 35, b = 58, c = 26, d = 45, R = 37; MS32, A = 5'-TCACCGGTGAATTCCACAGACACT-3', B = 5'-AAGCTCTCCATTTCCAGTTTCTGG-3', X = HpaI, Y = BglI, a = 181, b = 324, c = 10, d = 37, R = 29: pMS51, A = 5'-GATCAGCGAACTTCCTCTCGGCTC-3', B = 5'-TCCACATTGAGGACTGTGGGAAGC-3', X = DdeI, Y = HaeII, a = 117, b = 131, c = 30, d = 0, R = 25 + 33; p33.1, A = 5'-CTTTCTCCACGGATGGGATGCCAC-3', B = 5'-GCCGTGTCACCCACAAGCTTCTGG-3', x = DdeI, Y = RsaI, a = 6, b = 27, c = 0, d = 15, R = 62; p33.4, A = 5'-CCGGGCCAGACCCCAGCTGCTGAG-3' B = 5'-GCAGCATAGGGGCTGTCCTGGGCT-3', X = DdeI, Y = DdeI, a = 11, b = 96, c = 0, d = 2, R = 64: p33.6, A = 5'-TGTGAGTAGAGGAGACCTCACATT-3', B = 5'-AGGTGAGACATTACTCAATCCAAG-3', X = StyI, Y = DraIII, a = 14, b = 45, c = 10, d = 16, R = 37.

Figure 2

Amplification of MS32 minisatallite alleles by PCR. A, 0.1_g aliquots of DNA from CEPH individuals 2306, 10208, 133101 and 133304, which together contain 8 different MS32 alleles ranging from 1.1 to 17.9 kb, were pooled and amplified for 10-20 cycles in 10µl reactions containing 1 unit Taq polymerase plus flanking primers A and B. PCR products were separated by electrophoresis in a 1% agarose gel and detected by Southern blot hybridization with a minisatellite probe. Taq polymerase extension times at 70° were for 6, 15 or 30 minutes with (+) or without (-) addition of extra polymerase (1 unit) at the 10th cycle. H, 2µg of each CEPH DNA digested with AluI; AluI sites flanking MS32 are located such that each AluI allele is 0.2 kb longer than its corresponding PCR product. Autoradiography was for 5 hours (cycles 10, 14) or 1 hour (17, 20) without an intensifier screen. B, effect of increasing concentration of Taq polymerase (a-c, 0.5, 1, 2 units respectively) on the efficiency of amplification of large alleles. The extension time at 70° was 15 minutes.

Figure 3

Efficiency of amplification of MS32 minisatellite alleles as a function of allele length, with PCR extension times of 6 minutes (0) or 15 minutes (0). The gain in product per amplification cycle was determined by scanning laser densitometry of tracks H, 6+ and 15+ of Figure 2, exposed to pre-flashed X-ray film without an intensifier screen. The mean estimates of gain per cycle determined up to cycle 10, from cycle 10 to 14 and from cycle 14 to 17 were in close agreement, indicating that the yield of PCR product is increasing exponentially at least up to cycle 17; the Figure shows the mean value of the three estimates of the gain for each allele.

Figure 4

Co-amplification of two minisatellites from single cell equivalents of human DNA. A, 60 or 6pg aliquots of DNA from blood from an individual heterozygous for alleles a, b at λMS32 and c, d at pMS51 were amplified for 25 cycles with 15 minutes extension times in the presence of primers A and B for both loci, followed by Southern blot hybridization analysis of amplification products. Low levels of allele a could be detected in three of the 6pg samples on prolonged autoradiographic exposure (arrows). B, analysis of spurious amplification products of MS32. Two 60pg aliquots of DNA were amplified for 30 cycles, followed by digestion with S1 nuclease (S1), BglI (B) or HpaI (H). BglI cleaves once in the flanking DNA, between the MS32 minisatellite and primer B, and removes 311bp of flanking DNA. HpaI cleaves between primer A and the minisatellite, removing 195bp of flanking DNA (see Figure 1 legend).

Figure 5

Co-amplification of six different human minisatellites by PCR. A, amplification of 10ng (first four lanes) or 1ng DNA (last two lanes) from individual 1 for 15 or 18 cycles respectively, using a cocktail of primers A and B for ministatellites pλg3, MS32, pMS51, 33.1, 33.4 and 33.6. PCR products were detected by Southern blot hybridization with a cocktail of all six $^{32}$P-labelled minisatellite probes. The individual tested had been previously characterised at all six loci separately, which enabled all hybridizing DNA fragments to be assigned as shown; this individual is heterozygous at all six loci. These DNA fingerprints are from three separate experiments. Note that 33.4 has failed to amplify in the last tract. B, DNA fingerprints of 8 unrelated individuals (2-9.) following amplification of 1ng samples of DNA for 18 cycles. C, DNA fingerprints of 3-generation family (CEPH kindred 1435), following amplification of long DNA for 15 cycles. Three bands, corresponding to alleles of 33.4 and pMS51, failed to amplify in individual 12, as shown by a second analysis of this family (first tract, bands marked with an asterisk). In all experiments, PCR products were digested with S1 nuclease (see Materials and Methods) prior to gel electrophoresis, to reduce background labelling. DNA-free controls in all experiments were consistently blank (not shown).

Figure 6

Variability of DNA fingerprints producted by co-amplifying six minisatellites simultaneously by PCR. 1ng samples of DNA from 21 unrelated individuals were analysed as described in Figure 5. A, variation in the number of resolvable DNA fragments per individual. The mean number of bands resolved per differences seen between the DNA fingerprint of pairs of unrelated individuals, based on 29 independent pairwise comparisons. The number of discordant bands is the total number of bands not shared by the two individuals being compared. The theoretical maximum number of discordancies with 6 loci is 24, and the observed mean is 10.8 ± 2.8 (S.D.). The distribution of discordancies approximates to a Poisson distribution with this mean (dots).

Figure 7

Amplification of minisatellites from single human cells. A, samples containing 0, 1 or 3 small lymphocytes (from the individual analysed in Figure 5A) were lysed either with proteinase K plus SDS or by heating in water, followed by co-amplification with primers for MS32, pMS51, 33.1, 33.4 and 33.6 for 27 cycles. PCR products were Southern blot hybridized sequentially with each of the five minisatellite probes. B, amplification products of single buccal cells, analysed following lysis with proteinase K plus SDS and PCR as above. Cells from two individuals, a and b were tested; b is homozygous at pMS51, 33.1 and 33.6. 0, no cell control. Spurious PCR products are indicated with arrows.

Figure 8

Structure of plasmid clone pMS228. Boxed regions represent minisatellite arrays. There are two distinct minisatellite regions discernible; 228A detects the more strongly hybridising bands and 228B the fainter bands on high stringency hybridisation of human DNA.

Figure 9

Direct detection of amplified MS32 minisatellite alleles by electrophoresis on ethidium-stained agarose gels, following 30 cycles of PCR on 0.4µg human genomic DNA. M = marker DNA (0.5µg DNA x Hind III + 0.2µg 0x174 DNA x HaeIII). Individual 1 is heterozygous for the 4.5 kb allele detected on the gel and a larger allele (7 kb) which amplifies much less efficiently and cannot therefore be detected. Individual 2 is heterozygous for 3.7 and 4.6 kb alleles, both of which are detectable. The sizes of the amplified alleles correspond to the predicted sized determined by conventional Southern blot analysis of genomic DNA from these individuals. Note the presence of an additional complex set of low molecular weight PCR products, corresponding to collapsed minisatellite alleles. 0 = no DNA control.

Figure 10

MVR map of the smaller MS32 allele in CEPH individual 10202. A, Hinfl (F) and HaeIII (H) partial digest products of the PCR amplified allele, following cleavage with EcoRI and end-labelling at the EcoRI site. B, the complementary pattern from the same allele end-labelled at the ClaI site. C, map of Hinfl and HaeIII cleavage sites in the allele. The locations of PCR primers A and B are shown.

Figure 11

Strategy for mapping the location of variant repeat units within an MS32 minisatellite allele. A; location of PCR primers in relation to the minisatellite repeats (open boxes) and surrounding retroviral LTR homologue (filled box). Restriction sites for Hinfi = I (F), HaeIII (H) and Sau3A (S) are also shown. Primer sequences are A, 5'-TCACCGGTGAATTCCACAGACACT-3'; B, 5'-AAGCTCTCCATTTCCAGTTTCTGG-3'; C, 5'-CTTCCTCGTTCTCCTCAGCCCTAG-3'; D, 5'CGACTCGCAGATGGAGCAATGGCC-3'. Derivatives C1 and D1 with a 5' extension TCACCGGTGAATTC- containing an efficiently-cleaved EcoRI site (underlined) were used to generate PCR products with a unique EcoRI site suitable for end-labelling and mapping. B, internal mapping data on a small MS32 allele from CEPH individual 10208. This allele was amplified from genomic DNA using primers C1 plus D (left) or C plus D1 (right), end-labelled at the EcoRI site and partially digested with HinfI (F) or HaeIII (H), followed by agarose gel electrophoresis and autoradiography. The distribution of internal minisatellite repeat units in this allele cleaved or not cleaved by HaeIII is shown in (A).

## Figure 12

MVR maps of MS32 alleles sampled from various populations and aligned to shown map similarities. Each allele is coded according to whether each repeat unit is cleaved (a, A) or not cleaved, (t, T) by HaeIII. Each allele is given a serial number (1-32) in order of allele length together with population origin (A, Amish; E, English, F, French; M, Mormon; V, Venezuelan) and number of repeat units. High order tandem repetitions in the MVR maps are indicated below alleles by --->. MVR map regions common to more than one allele were identified by dot matrix comparisons of all pairwise combinations of alleles. Gaps (-) were introduced to optimize alignments. Four common 5' haplotypes (1, 2A, 2B, 3) were so identified. Haplotype 1 (uppercase) extends for 20-21 repeat units. Haplotype 2A (uppercase) is more variable in length. Haplotype 2b consists of the first 15 repeats of haplotype 2A (uppercase) followed by a variable number of additional repeat units (lowercase underlined). The short and provisionally identified haplotype 3 (uppercase) appears to be preferentially associated with alleles containing mainly a-type repeat units. Three remaining alleles "others" show no significant similarity to alleles classifiable into the 4 main 5' haplotypic groups. Matches between identical (3, 4) and very similar (24, 25, 26) alleles are indicated by vertical lines.

## Figure 13

Amplification of single molecule mutant MS32 alleles from size-fractionated sperm DNA. A, fidelity and efficiency of single molecule PCR. 30pg aliquots of a Sau3A plus MboI digest of sperm DNA from an individual heterozygous for alleles 31 and 32 (Figure 3) were amplified for 28 cycles using PCR primers A plus B (Figure 1A). PCR products were electrophoresed through a 0.8% agarose gel and Southern blot hybridized with a $^{32}$P-labelled MS32 minisatellite probe. 28 out of 40 duplicate PCR reactions showed PCR products from both alleles. From the poisson distribution, this indicates a mean of 1.8 successful amplification events per allele per reaction, compared with a mean input of 5 molecules of each allele. Thus 36% of input MS32 molecules give a PCR signal. B, single molecule amplification events from sperm DNA digested with Sau3A plus MboI and size-fractionated by gel electrophoresis. Multiple aliquots of DNA from fractions S1-S5 each containing respectively DNA from the equivalent of 0.03, 0.15, 0.6, 1.2 and 1.0 _g total sperm DNA were amplified for 25 cycles using PCR primers A plus B. PCR products were detected by Southern blot hybridization with an MS32 minisatellite probe. H, 30pg unfractionated sperm DNA. The size ranges of each fraction is shown. C, mutant minisatellites in (B) re-amplified using the nested PCR primers C1 plus D (Figure 11 legend) for 25-28 cycles followed by agarose gel electrophoresis and staining with ethidium bromide.

## Claims

1. A method of characterising a test sample of genomic DNA by reference to one or more controls, which method comprises amplifying the minisatellite sequence at at least one informative locus in the test sample by

(i) hybridising the test sample with primer in respect of each informative locus to be amplified, the primer being hybridisable to a single strand of the test sample at a region which flanks the minisatellite sequence of the informative locus to be amplified under conditions such that an extension product of the primer is synthesised which is complementary to and spans the said minisatellite sequence of the strand of the test sample;

(ii) separating the extension product so formed from the template on which it was synthesized to yield single stranded molecules;

(iii) if required hybridising the primer of step (i) with single stranded molecules obtained according to step (ii) under conditions such that a primer extension product is synthesised from the template of at least one of the single stranded molecules obtained according to step (ii), and

(iv) detecting the amplification products and comparing them with one or more controls;

the method being effected such that sufficient of the desired extension product is generated to be detectable but such that the yield of extension product is inadequate to permit substantial out-of-register hybridisation between complementary minisatellite template strands.

2. A method as claimed in claim 1 wherein the test sample of genomic DNA is subjected to restriction prior to amplification and only one primer is used in respect of each informative locus to be amplified.

3. A method as claimed in claim 1 which comprises

(i) hybridising the test sample with two primers in respect of each informative locus to be amplified, each primer being hybridisable to single strands of the test sample at a region which flanks the minisatellite sequence of the informative locus to be amplified under conditions such that an extension product of each primer is synthesised which is complementary to and spans the said minisatellite sequence of each strand of the test sample whereby the extension product synthesised from one primer, when it is separated from its complement, can serve as a template for synthesis of the extension product of the other primer;

(ii) separating the extension product so formed from the template on which it was synthesised to yield single stranded molecules;

(iii) if required hybridising the primers of step (i) with the single stranded molecules obtained according to step (ii) under conditions such that a primer extension product is synthesised from the template of each of the single stranded molecules obtained according to step (ii); and

(iv) detecting the amplification products and comparing them with one or more controls;

the method being effected such that sufficient of the desired extension product is generated to be detectable but such that the yield of extension product is inadequate to permit substantial out-of-register hybridisation between complementary minisatellite template strands.

4. A method as claimed in any one of the previous claims wherein alleles of an informative locus in the test sample are of up to 15 kilobases.

5. A method as claimed in any one of the previous claims which includes the use of at least one enzyme which specifically digests or degrades single stranded DNA whilst leaving double stranded DNA intact, whereby to alleviate the formation of aspecific amplification products.

6. A method as claimed in any one of the previous claims wherein amplification is effected in buffer of reduced ionic strength and at an elevated annealing temperature whereby to alleviate mispriming.

7. A method as claimed in any one of the previous claims wherein more than one informative locus is amplified simultaneously.

8. A method as claimed in any one of the previous claims for the characterisation of one molecule of an informative locus in a genomic DNA test sample.

9. A polynucleotide hybridisable to a single strand of a test sample of genomic DNA at a region which flanks the minisatellite sequence of an informative locus so as to permit formation of an extension product which is complementary to and spans the said minisatellite sequence of the strand of the test sample and wherein the informative locus is selected from any one of the following: MS1, MS29, MS31A and MS31B, MS32, MS43A and MS43B, MS51, MS228 and MS228B.

10. A polynucleotide extension product prepared from a polynucleotide primer hybridisable to a single strand of a genomic DNA test sample at a region which flanks the minisatellite sequence of an informative locus and wherein the polynucleotide extension product is complementary to and spans the said minisatellite sequence of the strand of the test sample.

11. A mixture which comprises multiple faithful copies of a polynucleotide extension product as claimed in claim 10 above.

12. A mixture as claimed in claim 11 above which comprises the products of at least 3 cycles of the amplification method as claimed in one of claims 1 - 7.

13. A kit comprising polynucleotide primer for amplifying the minisatellite sequence at at least one informative locus in a test sample of genomic DNA the primer being hybridisable to a single strand of the test sample at a region which flanks the minisatellite sequence of the informative locus to be amplified under conditions such that an extension product of the primer is synthesised which is complementary to and spans the said minisatellite sequence of the strand of the test sample; and the kit further comprising a control sample of DNA and instructions for use.

14. A kit as claimed in claim 13 which comprises two complementary flanking polynucleotide primers in respect of each informative locus.

Fig. 1.

EP 0 370 719 A2

# Fig.2.

Fig. 3.

*Fig. 4.*

EP 0 370 719 A2

Fig. 5.

EP 0 370 719 A2

# Fig. 6.

# Fig. 7.

# Fig. 8.

228 A                                    228B

1kb

# Fig.9.

EP 0 370 719 A2

*Fig. 10.*

A     B

F   H    F   H

C

EcoRI   F    F F F F F F F   F F F F F F F F   F F F F F F F F F

A    B                                     B

               H H   H H    H H H H    H     H   H H H H      ClaI

*Fig. 11.*

A

B

C1 + D          C + D1

# Fig. 12.

ALLELE

**5' haplotype 1**

6   F   58   TTTTTTAAATATTTTATTTATtataaatatataaaaaaaaaaaaaaaaattaaaaatat

12  F   81   TTTTTTAAATATTTTATTTATaattttaaaaattaaaaaaaaaaatataaaaaaaaaaaaattaaaaataaataaataaaaat

18  M   102  TTTTTTAAATATTTTATTTATtttaaaataaaattaattaatttaaataaaaatttaaatttaaatttaaatttaaatttataaataataaaaaaaaaattaat
                                                               ---->----->----->----->----->

19  E   139  TTTTTTAAATATTTTATTTAatatttaaaaaataaaaaaaaaaaaaaaaaaaaaaaaaaatttataaaatattattttttaaatattataaaatttaaaataaattaattaaat

ataaaaaataaaattttataaaaaat

**5' haplotype 2A**

30  E   149  TTTTTATATTTTATTAAAAAAAAAAAATAAAATTATAAAA-AAATAAAATTATAAAAAATAAAAAAAaaattaaaaaaaaaaaaaaaaaaaaatatatatataaatatatatata
                           --------------->  --------------->                                              ->->->->->

ataatttaatttattttaaaaaaaaataaataaattat

13  M   84   TTTT-ATATTTTATTAAAAAAAAAAAATAAAATTAaAAAAAAAAATAAAATTATAAAAAATAAAAAAAttaaaaaaaaaaaaaaat
                           --------------->  --------------->

9   E   74   TTTTTATATTTTATTAAAAAAAAAAAATAAAATTATAAAAAAAAATAAAATTATAAAAAATAAAAAAAaaaaaat
                           --------------->  --------------->

14  M   86   TTTTTATATTTTATTAAAAAAAAAAAATAAAATTATAAAAAAAAATAAAATTATAAAAAATAAAAttaaaataaaaaaaaaaaatat
                           --------------->--------------->

7   A   71   TTTTTATATTTTATTAAAAAAAAAAAATAAAATTATAAAAAAAAATAAAATTATAAAAAATAtttaataaaat
                           --------------->  --------------->

27  E   129  TTTTTATATTTTATTAAAAAAAAAAAATAAAATTATAAAAAAtataaaaaaataaaaaaaaaaattaaatatatatataaattaattattatataataaaataaataaataataata
                                                            ->->->->->

taaaaattaaatatt

**5' haplotype 2B**

16  F   99   TTTTTATATTTTATTtaaaaaaaaaattaaattataaaaaatatataaaatataaaaaaaaa--aaaaaaaaattaaaaaaataaaataaaattttaaaaat

20  E   104  TTTTTATATTTTATTtaaaaaaaaaattaaattataaaaaatatataaaatataaaaaaaaaaaaaaaaaaaattaaaaaaataaaataaaattttttaaaaatat

22  E   117  TTTTTATATTTTATTtaaaaaaaaaattaaattataaaaaataaataaaatataaaaaaaaa------aaaaaataaaaaaaataaaataa-attttaaatttaaaattaaaaa

taattaaaaaat

23  E   119  TTTTTATATTTTATTtaaaaaaaaaattaaattataaaaaataaataaaatataaaaaaaaa------aaaaaataaaaaaaataaaataa-attttaaaaaatataaaataataa

aaaaaaaaataatt

26  E   122  TTTTTATATTTTATTtaaaaaaaaaattaaattataaaaaaaaatataaaaaaaataaaaaaaaaaataaaaataataaaaaaaaaaaaaaatatatataataaaatatataaataaa
              ||||||||||||||||||||||||||||||||||||||||||||||||||||||||||||||||||||||||||||||||||||||||||||||||||||| |||||||||||||||||||||||||
              aaaaaaaat
              || ||||||

25  E   121  TTTTTATATTTTATTtaaaaaaaaaaattaaatttataaaaaaaaaatataaaaaaaaataaaaaaaaaaataaaaataataaaaaaaaaaaataaatatataataaaaatatataaataa
              |||||||||||||||||||||||||||||||||||||||| |||||||||||||||||||||||||||||||||||||||||||||||||||||||||||||||||||||||||||||||||||
              aaa-aaaaat
              ||| ||||||

# Fig. 12 (cont.)

24 E 120 TTTTTATATTTTATTtaaaaaaaaattaaattataaaaaaaaaaaataaaaaaaataaaaaaaaaataaaaataataaaaaaaaaaataaatatataataaaatatataaataa
aaa--aaaat

31 E 152 TTTTTATATTTTATTtaaaaaaaaattaaattataaaaaaaaaatataaaaatataaaaaaaaaaaaaaaaaaataaaaaaaaaaaaaaaaaatataaatatatatatataataattt
aaaaaaaateaaaaaatataataatttaaaaatataataat →-›-›-›-›

5 E 54 TTTTTATATTTTATTtaaaaaaaaaattaaattataaaaaaaaataaaaaaaaaaat

homogeneous alleles, 5' haplotype 3

11 E 80 TTAATataaaaaaaaaaaaaaaaaaaaaaaaaaaaaaaaaaaaaaaaaaaaaaaaaaaaaaaaataaaaataaaaataat

10 M 77 TTAATaaaaaaaaaaaaaaaaaaaaaaaaaaaaaaaaaaaaaaaaaaaaaaaaaaaaaaaaaaaaaaaaaaaaaaaatt

15 E 96 TTAATaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaatt

19 M 104 TTAATaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaattttat

17 M 102 TTAAaaaaaaaaaaaaaaaaaaaaaaaaaaaaaaaaaaaaaaaaaaaaaaaaaaaaaaaaatttaaaaaaaaaaaatataaaattttataataaaaaaaataaaat

21 E 107 TTAAaaaaaaaaaaaaaaaaaaaaaaaaaaaaaaaaaaaaaaaaaaaaaaaaaaaaaaaaaaaaaaaataaaaaaaaaaatataaaaatataaaataaaaaaaat

28 E 132 TTAAaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaataaa
aaataaaatttttttaaaaaaaaaat

2 M 31 taaaaaaaaaaaaaaaaaaaaaaaaaaaatat

8 F 73 taaaaaaaaaaaaaaaaaaaaaaaaaaaaaaaaaaaaaaaaaaaatttaaaaaaaaaaaaaaaaaaaaaaaaaaat

32 E 161 taaaaaataaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaaa
aaataataaaattataaaaaaaaaataaaataaaataaaaaaaaataaaaaatatt

others

1 V 23 ttaataattaaattattataaat

3 E 39 ttttttttatattttttataaaaaaaaaaaaatttattaaat
|||||||||||||||||||||||||||||||||||||||||||
4 V 39 ttttttttatattttttataaaaaaaaaaaaatttattaaat

Fig.13.

EP 0 370 719 A2